# EUROPEAN PATENT SPECIFICATION

(11) **EP 2 869 717 B1**
(45) Date of publication and mention of the grant of the patent: **13.06.2018**
(21) Application number: 13816903.2
(22) Date of filing: 09.07.2013
(51) Int. Cl.: A61K 31/122, A61P 43/00

(54) **PREVENTION OF ALCOHOL REACTION WITH DIETARY SUPPLEMENTS**
PRÄVENTION VON ALKOHOLREAKTIONEN MIT NAHRUNGSZUSÄTZEN
PRÉVENTION D'UNE RÉACTION À L'ALCOOL AVEC DES COMPLÉMENTS DIÉTÉTIQUES

(30) Priority: 09.07.2012 US 201261669404 P; 13.03.2013 US 201361780612 P
(43) Date of publication of application: 13.05.2015
(73) Proprietor: Noglo LLC, San Francisco, CA 94103 (US)
(72) Inventor: Tolleth, Robert J., Millbrae, CA 94030 (US); Gordon, Spencer A., Ramona, CA 92065 (US)
(74) Representative: Wytenburg, Wilhelmus Johannes
(86) International application number: PCT/US2013/049783
(87) International publication number: WO 2014/011676

(56) References cited:
- WO-A1-2007/017139
- WO-A1-2012/120036
- WO-A2-2012/027603
- US-A1- 2001 033 881
- US-A1- 2007 202 215
- US-B1- 8 377 907
- Anonymous: "NoGlo Ingredients", Internet , 16 November 2012 (2012-11-16), XP002752542, Retrieved from the Internet: URL:http://gonoglo.com/noglo-ingredients/ [retrieved on 2015-11-25]
- Anonymous: "Drinkwel Multivitamin Review - The Cure for Hangovers", Internet , 5 April 2012 (2012-04-05), XP002752543, Retrieved from the Internet: URL:http://www.chipchick.com/2011/09/drink wel-revie.html [retrieved on 2015-11-25]
- Anonymous: "How to prevent the damaging effects of smoking, alcohol consumption, and air pollution", Internet , 5 April 2012 (2012-04-05), XP002752544, Retrieved from the Internet: URL:http://coenzyme-a.com/effects_of_smoki ng_alcohol_airpollution_article.html [retrieved on 2015-11-25]
- Anonymous: "Mega antioxidant tablets", Internet , 15 April 2012 (2012-04-15), XP002752545, Retrieved from the Internet: URL:http://www.drugs.com/drp/mega-antioxid ant-tablets.html [retrieved on 2015-11-25]
- KANE ET AL.: 'Ethanol elevates physiological all-trans-retinoic acid levels in select loci through altering retinoid metabolism in multiple loci: a potential mechanism of ethanol toxicity' THE FASEB JOURNAL vol. 24, no. 3, March 2010, pages 823 - 832, XP055185018

## Description

### TECHNICAL FIELD

The present invention relates to compositions for use by a subject to prevent the flush and other unpleasant symptoms that sometimes accompany the consumption of alcoholic beverages.

### BACKGROUND

The alcohol reaction, also known as the alcohol flush reaction, is an unpleasant condition which occurs in some individuals upon consumption of alcohol, even in relatively small amounts. The reaction is characterized by flushing of the facial skin, and sometimes other areas of the skin. The alcohol flush reaction may also include dizziness, nausea, stupor, headache, light-headedness, increased heart rate, and other symptoms.

The alcohol flush reaction occurs in individuals with lower rates of metabolism of acetaldehyde. Acetaldehyde is metabolized by acetaldehyde dehydrogenase-2 (ALDH2; also known as mitochondrial aldehyde dehydrogenase; human precursor Swiss-Prot P05091), a liver enzyme involved in the metabolism of ethanol, which oxidizes acetaldehyde to acetic acid. Approximately 1 billion people, including up to about 50% of people of Chinese, Japanese and Korean ethnicity, have a point mutation resulting in a less active version of ALDH2 (ALDH2*2, where a glutamic acid residue is mutated to lysine, i.e., ALDH2 E487K; the residue numbering is based on the mature protein; see, e.g., Steinmetz et al., Structure 5: 701-11, 1997), which functions at about 8% of the wild-type form. The enzyme is normally a homotetramer. However, if an individual carries one mutated allele, then he or she will present the mutant phenotype due to the formation of heterotetramers. The mutation in ALDH2 is at an amino acid residue located at the binding site for nicotinamide adenine dinucleotide (NAD), an enzyme cofactor required for the conversion of acetaldehyde to acetic acid; in the process, NAD is converted to NADH. The lowered activity of this enzyme results in an accumulation of acetaldehyde in the blood when consuming alcohol. Certain individuals, especially those of East Asian descent, may also have a highly active variant of alcohol dehydrogenase (ADH1B; also known as alcohol dehydrogenase 1B; human precursor Swiss-Prot P00325) caused by a R47H mutation (again, this residue numbering is based on the mature protein), leading to more rapid accumulation of acetaldehyde (Peng et al., BMC Evolutionary Biology 2010, 10:15). Individuals with both the "fast" variant of alcohol dehydrogenase (which produces acetaldehyde from alcohol) and the "slow" variant of acetaldehyde dehydrogenase (which eliminates acetaldehyde) can be strongly affected by the alcohol flush reaction.

Acetaldehyde is 30 times as toxic as ethanol and is a known carcinogen. In addition to causing inflammation and flushing of the skin, acetaldehyde can cause organ damage. There is evidence demonstrating that individuals with the variant ALDH2 enzyme are at a significantly increased risk of gastric and esophageal cancers, liver cirrhosis and failure, and even Alzheimer's disease if they consume alcohol with any regularity. This is shown to be due to the accumulation of acetaldehyde. Individuals that demonstrate the flush response to alcoholic beverages and drink moderately are at a much higher risk of esophageal cancer than even heavy drinkers who do not experience the flush reaction. In addition, drinkers that flush may also experience dizziness and nausea after just one drink.

The health risks associated with drinking and flushing are not well understood by the general public. The majority of research on the ALDH2 mutation and alcohol related diseases has been done in Japan, and the results of this research are not as widely disseminated in other countries, including the United States, which in 2009 had an estimated 4 million residents of Chinese descent, 1.6 million residents of Korean descent, 1.3 million residents of Japanese descent, and several million additional residents from other East Asian countries. While awareness of the health risks associated with the alcohol flush reaction is increasing in Japan and China, people continue to drink regularly despite these known long term risks.

Thus, there is a need for compositions and methods for prevention or reduction of the alcohol flush reaction, both for the short-term comfort of the alcohol consumer, and for the potential to reduce the long-term health risks associated with consumption of alcoholic beverages by persons demonstrating the alcohol flush reaction.

WO 2012/120036 A1 (Gerdes Roman - 13 September 2012) describes a pharmaceutical composition for reducing and/or preventing the adverse effects of alcohol consumption.

US 8377907 B1 (Halamicek III, William A - 19 February 2013) describes compositions for treating alcohol hangover.

"NoGlo Ingredients" (16 November 2012 - retrieved from http://web.archive.org/ web/ 20121116062353/http://gonoglo.com/noglo-ingredients on 25 November 2015) describes an anti-hangover, anti-flush supplement.

"Drinkwel Multivitamin Review - The Cure for Hangovers" (21 September 2011 - retrieved from http://www.chipchick.com/2011/09/drinkwel-revie.html on 25 November 2015) describes pills which "severely reduce, or eliminate, the hangover".

"How to prevent the damaging effects of smoking, alcohol consumption, and air pollution" (5 April 2012 - retrieved from http://coenzyme-a.com/effects_of_smoking_alcohol_ airpollution_article.html on 25 November 2015) describes the common sources of acetaldehyde and its damaging effects on brain structure and function and provides a list of possible nutrient levels that may offer protection against chronic aldehyde toxicity, in an amount per day.

"Mega antioxidant tablets" (15 April 2012 - retrieved from http://www.drugs.com/ drp/mega-antioxidant-tablets.html on 25 November 2015) describes tablets that contain essential vitamins and antioxidants at levels substantially higher than the recommended daily allowance (RDA) amounts.

WO 2007/017139 A1 (Tima Foundation -15 February 2007) describes a composition for moderating alcohol metabolism and for reducing the risk of alcohol induced diseases.

WO 2012/027603 A2 (Clarity Products LLC - 1 March 2012) describes a therapeutic consumable composition for the reduction of facial flush in response to consumption of alcohol in persons with deficient activity of the aldehyde dehydrogenase gene ALDH2.

Kane et al., 2010, The FASEB Journal, Vol 24, No. 3, pp. 823-832, describes a study of the effects of ethanol on all-trans-retinoic acid (atRA) concentration in different tissues (hippocampus, liver, etc.) in mice during normal vitamin A nutriture and suggested that elevated levels of atRA contribute to ethanol-induced disorders such as foetal alcohol syndrome (FAS).

US 2007/202215 A1 (Lak, Z. S. - 30 August 2007) describes a dietary nutritional supplement for humans who consume moderate to large amounts of alcohol.

US 2001/033881 A1 (Fuchs et al. - 25 October 2001) describes a fructose-containing refreshing drink composition for increasing the alcohol degradation capacity of the body.

### SUMMARY OF THE INVENTION

The present invention relates to a composition for use by a subject to prevent or reduce the alcohol flush reaction, said composition comprising:
(a) an agent selected from nicotinamide, nicotinamide riboside, niacin, and oxaloacetate; and
(b) an agent selected from N-acetyl-cysteine, Vitamin C, alpha lipoic acid, L-cysteine, glutathione, glycine, L-glutamic acid, adenosylmethionine, and conjugated linoleic acid; and
(c) an agent selected from pantothenic acid, pantethine, pantetheine, 4'-phosphopantothenate, and 4'-phosphopantetheine;
wherein the composition is administered daily for at least 30 days prior to consumption of alcohol by the subject;
wherein said agents are present in the composition in an amount sufficient to prevent or reduce the alcohol flush reaction in the subject.

In one embodiment, the composition further comprises an agent which is selected from Vitamin C, Vitamin E, and alpha-lipoic acid.

In one embodiment, the composition further comprises alpha-lipoic acid.

In one embodiment, the composition further comprises an agent selected from beta-carotene and a retinol ester.

In one embodiment, the agent is a retinol ester selected from retinyl arachidonate, retinyl stearate, retinyl oleate, retinyl linoleate, retinyl heptadecanoate, retinyl palmitate, retinyl myristate, retinyl laurate, retinyl caprylate, and retinyl acetate.

In one embodiment, the agent is beta-carotene.

In one embodiment, the composition further comprises thiamine.

In one embodiment, the composition comprises:
(i) between 300 mg and 1.5 g nicotinamide,
(ii) between 10 mg and 500 mg pantothenic acid or a salt thereof, and
(iii) between 100 mg and 600 mg N-acetylcysteine,
and an excipient acceptable for daily oral administration.

In one embodiment, the composition further comprises vitamin C in an amount between 100 mg and 500 mg.

In one embodiment, the composition further comprises alpha-lipoic acid in an amount between 50 mg and 250 mg.

In one embodiment, the composition further comprises beta-carotene in an amount between 100 IU and 15000 IU.

In one embodiment, the composition further comprises thiamine.

In one embodiment, the composition further comprises thiamine in an amount between 2.5 mg and 10 mg.

In one embodiment, the composition further comprises one or more of vitamin A, vitamin C, thiamine, and α-lipoic acid.

In one embodiment, the composition further comprises one or more of:
1250 IU to 2500 IU vitamin A,
75 mg to 150 mg vitamin C,
2.5 to 10 mg thiamine, and
62 to 125 mg α-lipoic acid.

In one embodiment, the composition further comprises each of vitamin A, vitamin C, thiamine, and α-lipoic acid.

In one embodiment, the composition is provided as a tablet or a capsule.

In one embodiment, the composition is provided in a food or in a beverage.

### DESCRIPTION

Described herein are compositions and methods for prevention or reduction of the alcohol flush reaction in a subject.

Also described herein is a composition for use by a subject to prevent or reduce the alcohol flush reaction and/or to reduce acetaldehyde in tissues and body fluids (e.g., liver, central nervous system, blood, saliva, etc.). The composition comprises an agent capable of increasing intracellular or intramitochondrial concentration of NAD in the subject, and an agent capable of increasing glutathione concentration in the subject, where the agents are present in the composition in an amount sufficient to prevent or reduce the alcohol flush reaction in the subject. Also as described herein, the composition can further comprise one or more of the following:

an agent capable of increasing the level of Coenzyme A in the subject;

an agent which is an anti-oxidant;

alpha-lipoic acid or a precursor to alpha-lipoic acid;

an agent capable of increasing the level of Coenzyme A in the subject and an anti-oxidant;

an agent capable of increasing the level of Coenzyme A in the subject and alpha-lipoic acid or a precursor to alpha-lipoic acid;

an anti-oxidant and alpha-lipoic acid or a precursor to alpha-lipoic acid; or

an agent capable of increasing the level of Coenzyme A in the subject, an anti-oxidant, and alpha-lipoic acid or a precursor to alpha-lipoic acid. This list is not meant to be limiting.

Also described herein is a composition for use by a subject to prevent or reduce the alcohol flush reaction and/or to reduce acetaldehyde in tissues and body fluids, where the composition comprises an agent capable of increasing intracellular or intramitochondrial concentration of NAD in the subject. Also described herein is a composition for use by a subject to prevent or reduce the alcohol flush reaction, where the composition comprises an agent capable of increasing glutathione concentration in the subject. Also described herein is a composition for use by a subject to prevent or reduce the alcohol flush reaction, where the composition comprises an agent capable of increasing intracellular Coenzyme A concentration in the subject. In these embodiments, the agent is present in the composition in an amount sufficient to prevent or reduce the alcohol flush reaction in the subject. The agent can be administered to the subject in a daily dosage for at least one week prior to consumption of alcohol. The daily dosage can be administered in a single dosage per day, or in multiple divided dosages per day.

Also described herein is a composition for use by a subject to prevent or reduce the alcohol flush reaction and/or to reduce acetaldehyde in tissues and body fluids. The composition can be described as an anti-alcohol flush reaction supplement. The supplement or composition comprises an agent capable of increasing intracellular or intramitochondrial concentration of NAD in the subject; an agent capable of increasing glutathione concentration in the subject; an agent which is an anti-oxidant; an agent which is alpha-lipoic acid or a precursor to alpha-lipoic acid; and an agent capable of increasing the level of Coenzyme A in the subject. The agents are present in the supplement or composition in an amount or amounts effective to prevent or reduce the alcohol flush reaction in the subject. In other embodiments, the agents are present in the supplement or composition in an amount or amounts effective to prevent the alcohol flush reaction in the subject. In other embodiments, the agents are present in the supplement or composition in an amount or amounts effective to reduce the alcohol reaction in the subject.

As described herein, the agent capable of increasing intracellular or intramitochondrial concentration of NAD in the subject can be nicotinamide, niacin, nicotinamide riboside, or oxaloacetate. If nicotinamide is used, the amount of nicotinamide in the supplement or composition can range from about 500 mg to about 1,500 mg, or from about 750 mg to about 1,250 mg. In another embodiment, the amount of nicotinamide in the supplement or composition is about 1000 mg. In another embodiment, the amount of nicotinamide in the supplement or composition is about 800 mg. If niacin is used, the amount of niacin in the supplement or composition can range from about 500 mg to about 1,500 mg, or from about 750 mg to about 1,250 mg. In another embodiment, the amount of niacin in the supplement or composition is about 1000 mg. In another embodiment, the amount of niacin in the supplement or composition is about 800 mg. If nicotinamide riboside is used, the amount of nicotinamide riboside in the supplement or composition can range from about 1,000 mg to about 3,200 mg, or from about 1,500 mg to about 2,600 mg. In another embodiment, the amount of nicotinamide riboside in the supplement or composition is about 2,100 mg. In another embodiment, the amount of nicotinamide riboside in the supplement or composition is about 1,700 mg.

As described herein, the agent capable of increasing glutathione concentration in the subject can be N-acetyl cysteine, L-cysteine, Vitamin C, alpha lipoic acid, glutathione, glycine, L-glutamic acid, adenosylmethionine, or conjugated linoleic acid. If N-acetyl cysteine is used, the amount in the supplement or composition can range from about 200 mg to about 600 mg, or from about 300 mg to about 500 mg. In another embodiment, the amount of N-acetyl cysteine in the supplement or composition is about 400 mg. In another embodiment, the amount of N-acetyl cysteine in the supplement or composition is about 300 mg. If L-cysteine is used, the amount in the supplement or composition can range from about 200 mg to about 600 mg, or from about 300 mg to about 500 mg. In another embodiment, the amount of L-cysteine in the supplement or composition is about 400 mg. In another embodiment, the amount of L-cysteine in the supplement or composition is about 300 mg.

As described herein, the agent used as the anti-oxidant can be Vitamin C, Vitamin E, or alpha-lipoic acid. If Vitamin C is used, the amount in the supplement or composition can range from about 100 mg to about 500 mg, or from about 200 mg to about 400 mg. In another embodiment, the amount of Vitamin C in the supplement or composition is about 300 mg. In another embodiment, the amount of Vitamin C in the supplement or composition is about 150 mg.

As described herein, the amount of alpha lipoic acid in the supplement or composition, when a separate anti-oxidant is used in addition to alpha lipoic acid, can range from about 50 mg to about 250 mg, or from about 100 mg to about 200 mg. In one embodiment, the amount of alpha-lipoic acid in the supplement or composition is about 150 mg. In one embodiment, the amount of alpha-lipoic acid in the supplement or composition is about 100 mg. If alpha lipoic acid is also used as the anti-oxidant, the amount of alpha lipoic acid in the supplement or composition can range from about 100 mg to about 500 mg, from about 200 mg to about 400 mg, or in another embodiment is about 300 mg, or in another embodiment is about 200 mg.

As described herein, the agent capable of increasing the level of Coenzyme A in the subject can be pantothenic acid, pantothenate, panthenol, pantethine, pantetheine, 4'-phosphopantothenate, 4'-phosphopantetheine, N-acetyl-cysteine, or L-cysteine. If pantothenic acid is used, the amount of pantothenic acid in the supplement or composition can range from about 50 mg to about 250 mg, or from about 100 mg to about 200 mg. In another embodiment, the amount of pantothenic acid in the supplement or composition is about 150 mg. In another embodiment, the amount of pantothenic acid in the supplement or composition is about 100 mg. If pantothenate is used, the amount of pantothenate in the supplement or composition can range from about 50 mg to about 250 mg, or from about 100 mg to about 200 mg. In another embodiment, the amount of pantothenate in the supplement or composition is about 150 mg. In another embodiment, the amount of pantothenate in the supplement or composition is about 100 mg. If panthenol is used, the amount of panthenol in the supplement or composition can range from about 50 mg to about 250 mg, or from about 100 mg to about 200 mg. In another embodiment, the amount of panthenol in the supplement or composition is about 150 mg. In another embodiment, the amount of panthenol in the supplement or composition is about 100 mg.

As described herein are compositions which comprise (i) between 300 mg and 1.5 g nicotinamide, (ii) between 10 mg and 500 mg pantothenic acid or a salt thereof, and between 100 mg and 600 mg N-acetylcysteine; with preferred compositions comprising about 700 mg nicotinamide, about 25 mg pantothenic acid, and about 300 mg N-acetylcysteine, wherein the term "about" refers to +/- 20% of each quantity. By way of example, treatment can comprise a daily dosage of about 700 mg nicotinamide, 25 mg pantothenic acid, and 300 mg N-acetylcysteine. These compositions can further comprise one or more of vitamin A, vitamin C, thiamine, and α-lipoic acid. By way of example only, the compositions comprise one or more of, and preferably each of 1250 IU to 2500 IU vitamin A, 75 mg to 150 mg vitamin C, 2.5 to 10 mg thiamine, and 62 to 125 mg α-lipoic acid. This list is not meant to be limiting.

As noted herein, such daily dosages can be taken as a single administration, or in 2, 3, 4, 5 or more individual administrations. The skilled artisan will understand that the components of the composition may be provided as various salt or prodrug forms. By way of example only, pantothenic acid may be provided in the form of calcium pantothenate.

Preferred are compositions which comprise one or more pharmaceutical excipients acceptable for oral or enteral administration. The compositions may be provided as a tablet or capsule; as a beverage; as a food; etc.

As described herein is the establishment of a daily dose over a period of time, e.g. 3 days, 5 days, 10 days, 14 days, 21 days, 30 days, or longer, and administering a supplemental dosage between 10 minutes and 1 hour prior to consumption of ethanol by the human adult.

As described herein, the levels of the compositions described herein can reduce average peak blood levels of acetaldehyde in a population of human adults having an E487K mutation in mitochondrial aldehyde dehydrogenase and/or a R47H mutation in alcohol dehydrogenase 1B following ingestion of 0.5 g ethanol/kg body wt by at least 5%, more preferably 10%, still more preferably 20%, yet more preferably 40%, and most preferably 50% or more relative to average peak blood levels in a corresponding untreated population.

Also disclosed herein is a method of preventing or reducing at least one symptom of the alcohol flush reaction in a subject, by administering the supplement or composition as described herein. The dosage of the supplement or composition can be administered to the subject daily, either in a single dose per day, or in multiple divided doses per day. The composition is administered prior to the consumption of alcohol, and can be administered daily for a period of time such as at least seven days prior to consumption of alcohol by the subject, at least 14 days prior to consumption of alcohol by the subject, or at least 30 days prior to consumption of alcohol by the subject.

Also disclosed herein is a method of preventing or reducing at least one symptom of the alcohol flush reaction in a subject, by administering to the subject at least one agent selected from the group consisting of an agent capable of increasing intracellular or intramitochondrial concentration of NAD in the subject, an agent capable of increasing glutathione concentration in the subject, and an agent capable of increasing cellular Coenzyme A concentrations in the subject, where the at least one agent is administered in an amount sufficient to prevent or reduce the alcohol flush reaction in the subject. The dosage of the agent or agents can be administered to the subject daily, either in a single dose per day, or in multiple divided doses per day. The composition is administered prior to the consumption of alcohol, and can be administered daily for a period of time such as at least seven days prior to consumption of alcohol by the subject, at least 14 days prior to consumption of alcohol by the subject, or at least 30 days prior to consumption of alcohol by the subject.

As described herein, the symptom or symptoms to be prevented or reduced are selected from facial flushing, hangover, headache, stupor, dizziness, nausea, accumulation of acetaldehyde in the blood (i.e., increased levels of blood acetaldehyde), or increased heart rate. In some embodiments, the symptom to be reduced is facial flushing. In another embodiment, the symptom to be reduced is hangover. In another embodiment, the symptom to be reduced is headache. In another embodiment, the symptom to be reduced is stupor. In another embodiment, the symptom to be reduced is dizziness. In another embodiment, the symptom to be reduced is nausea. In another embodiment, the symptom to be reduced is accumulation of acetaldehyde in the blood or increased levels of blood acetaldehyde. In another embodiment, the symptom to be reduced is increased heart rate. In another embodiment, the symptoms to be reduced are any combination of any or all of the foregoing symptoms. In some embodiments, the symptom to be prevented is facial flushing. In another embodiment, the symptom to be prevented is hangover. In another embodiment, the symptom to be prevented is headache. In another embodiment, the symptom to be prevented is stupor. In another embodiment, the symptom to be prevented is dizziness. In another embodiment, the symptom to be prevented is nausea. In another embodiment, the symptom to be prevented is accumulation of acetaldehyde in the blood. In another embodiment, the symptom to be prevented is increased heart rate. In another embodiment, the symptoms to be prevented are any combination of any or all of the foregoing symptoms.

Also disclosed herein is a method of reducing the accumulation of acetaldehyde in the blood after consumption of alcohol, by administering the supplement or composition as described herein. As described herein is a method of reducing the accumulation of acetaldehyde in the blood after consumption of alcohol, by administering the supplement or composition as described herein in an amount and duration sufficient to reduce the accumulation of acetaldehyde by about 5%, up to about 5%, or by at least about 5% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 10%, up to about 10%, or by at least about 10% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 15%, up to about 15%, or by at least about 15% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 20%, up to about 20%, or by at least about 20% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 25%, up to about 25%, or by at least about 25% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 50%, up to about 50%, or by at least about 50% from the blood concentration that would otherwise be reached without administration of the supplement or composition. As described herein is method of reducing the accumulation of acetaldehyde in the blood after consumption of alcohol, by administering the supplement or composition as described herein in an amount and duration sufficient to reduce the accumulation of acetaldehyde by about 75%, up to about 75%, or by at least about 75% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 90%, up to about 90%, or by at least about 90% from the blood concentration that would otherwise be reached without administration of the supplement or composition. As described herein is a method of reducing the accumulation of acetaldehyde in the blood after consumption of alcohol, by administering the supplement or composition as described herein in an amount and duration sufficient to reduce the accumulation of acetaldehyde by about 95%, up to about 95%, or by at least about 95% from the blood concentration that would otherwise be reached without administration of the supplement or composition.

Also disclosed herein is the use of any of the compositions described herein for prevention or reduction of at least one symptom of the alcohol flush reaction in a subject. Also disclosed herein is the use of any of the compositions described herein for the manufacture of a medicament for use in preventing or reducing at least one symptom of the alcohol flush reaction in a subject.

As described herein, in any of the embodiments of the compositions, supplements, methods, or uses described herein, the composition or supplement can further comprise an agent capable of raising the blood level of all-trans retinoic acid.

The features and embodiments disclosed herein can be combined and permuted in any desired manner. These and other features and embodiments will become evident upon reference to the remainder of this application, including the following detailed description.

Some embodiments described herein are recited as "comprising" or "comprises" with respect to their various elements. In alternative embodiments, those elements can be recited with the transitional phrase "consisting essentially of" or "consists essentially of" as applied to those elements. In further alternative embodiments, those elements can be recited with the transitional phrase "consisting of" or "consists of" as applied to those elements. Thus, for example, if a composition or method is disclosed herein as comprising A and B, the alternative embodiment for that composition or method of "consisting essentially of A and B" and the alternative embodiment for that composition or method of "consisting of A and B" are also considered to have been disclosed herein. Likewise, embodiments recited as "consisting essentially of" or "consisting of" with respect to their various elements can also be recited as "comprising" as applied to those elements. Finally, embodiments recited as "consisting essentially of" with respect to their various elements can also be recited as "consisting of" as applied to those elements, and embodiments recited as "consisting of" with respect to their various elements can also be recited as "consisting essentially of" as applied to those elements.

When a composition is described as "consisting essentially of" the listed components, the composition contains the components expressly listed, and may contain other components which do not substantially affect condition being treated. However, the composition either does not contain any other components which do substantially affect the condition being treated other than those components expressly listed; or, if the composition does contain extra components other than those listed which substantially affect the condition being treated, the composition does not contain a sufficient concentration or amount of those extra components to substantially affect the condition being treated. When a method is described as "consisting essentially of" the listed steps, the method contains the steps listed, and may contain other steps that do not substantially affect the condition being treated, but the method does not contain any other steps which substantially affect the condition being treated other than those steps expressly listed.

The compositions and methods described herein, including any embodiment as described herein, may be used alone or may be used in combination with other compositions and methods.

### DETAILED DESCRIPTION

Described herein are compositions which are combinations of dietary supplements or compositions useful for prevention or reduction of the alcohol flush reaction. Also disclosed herein are methods for use, including dosage regimens, of the compositions to prevent or reduce the alcohol flush reaction. As described herein, the supplement or composition may reduce the accumulation of acetaldehyde in the blood after consumption of alcoholic beverages.

A supplement or composition, as described herein, comprising the combined ingredients can be taken once a day, or in multiple divided dosages over a day, or can be taken as individual components over the course of a day. The supplements or compositions comprise vitamins, amino acids, anti-oxidants, and other dietary supplements. While not wishing to be limited by theory, it is hypothesized that use of the supplements or compositions described herein results in a protective buildup of compounds which reduces the reactivity of acetaldehyde and which enhances enzyme kinetics, causing an increased rate of breakdown of acetaldehyde. The ALDH2 E487K enzyme variant binds NAD less strongly than the variant with glutamic acid at position 487. Thus, it is hypothesized that increasing NAD concentration will enhance the binding of NAD to aldehyde dehydrogenase, increasing the rate of acetaldehyde dehydrogenation.

Acetaldehyde is oxidized to acetic acid by ALDH2. Acetic acid then binds to Coenzyme A to form Acetyl-CoA and enters the Citric Acid Cycle. While not wishing to be limited by theory, it is hypothesized that during alcohol metabolism in subjects with the ALDH2 mutation, there is a build-up of acetic acid, and Coenzyme A is greatly reduced, as it is not regenerated quickly enough to keep up with the rate of production of acetic acid. With an accumulation of acetic acid, ALDH2 is inhibited based on known enzyme kinetics, as an increase in product concentration will decrease the favorability of the forward reaction. Also described herein is daily supplementation of any compound(s) to increase cellular Coenzyme A concentrations to increase the rate of formation of Acetyl-CoA, therefore clearing acetic acid more quickly and pushing the ALDH2 reaction towards the product. This reduces the accumulation and exposure to acetaldehyde, and as a result, reduces or prevents the alcohol flush reaction.

### Definitions

A subject, individual, or patient is a mammal, preferably a human.

Alcohol as used herein refers to ethanol (ethyl alcohol), unless another alcohol is explicitly indicated.

Preventing the alcohol flush reaction is defined as preventing at least one symptom of the alcohol flush reaction, such as facial flushing, dizziness, nausea, increased heart rate, or other symptoms in a subject who would ordinarily experience such a reaction absent the use of the compositions and methods as described herein, or of preventing the buildup of acetaldehyde in the blood subsequent to alcohol consumption, that would occur absent the use of the compositions and methods as described herein. Reducing the alcohol flush reaction is defined as reducing the intensity of at least one symptom of the alcohol flush reaction, such as facial flushing, dizziness, nausea, increased heart rate, or other symptoms in a subject, or of reducing the buildup of acetaldehyde in the blood subsequent to alcohol consumption, as compared to the intensity of said symptoms in the subject absent the use of the compositions and methods described herein. Prevention or reduction of the symptoms of the alcohol flush reaction can be self-reported by the subject (that is, subjective), or reported or measured in the subject for symptoms amenable to observation by others (that is, objective).

The compounds described herein can occur and can be used as the neutral (non-salt) compounds; however, the description is intended to embrace all salts of the compounds described herein, as well as methods of using such salts of the compounds. In one embodiment, the salts of the compounds comprise pharmaceutically acceptable salts. Pharmaceutically acceptable salts are those salts which can be administered as drugs or pharmaceuticals to humans and/or animals and which, upon administration, retain at least some of the biological activity of the free compound (neutral compound or non-salt compound). The desired salt of a basic compound may be prepared by methods known to those of skill in the art by treating the compound with an acid. Examples of inorganic acids include, but are not limited to, hydrochloric acid, hydrobromic acid, sulfuric acid, nitric acid, and phosphoric acid. Examples of organic acids include, but are not limited to, formic acid, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, sulfonic acids, and salicylic acid. Salts of basic compounds with amino acids, such as aspartate salts and glutamate salts, can also be prepared. The desired salt of an acidic compound can be prepared by methods known to those of skill in the art by treating the compound with a base. Examples of inorganic salts of acid compounds include, but are not limited to, alkali metal and alkaline earth salts, such as sodium salts, potassium salts, magnesium salts, and calcium salts; ammonium salts; and aluminum salts. Examples of organic salts of acid compounds include, but are not limited to, procaine, dibenzylamine, N-ethylpiperidine, N,N-dibenzylethylenediamine, and triethylamine salts. Salts of acidic compounds with amino acids, such as lysine salts, can also be prepared. When salts of compounds are used, the weight of the compound(s) used in the compositions and supplements described herein can be adjusted to take into account the additional weight contributed due to the salt form.

By "supplement" is meant a composition that is taken in addition to the ordinary diet of an individual, subject, or patient.

### Measurement and quantitation of symptoms

The various symptoms of the alcohol flush reaction can be self-reported by the subject either qualitatively (for example, flushing or not flushing) or quantitatively (for example, on a scale of one to ten for any of the symptoms; hangovers can be measured using standard scales used to describe pain in clinical settings, such as the Wong-Baker FACES Pain Rating Scale or the 0-10 Numeric Pain Rating Scale described by McCaffery M. and Casero, C. in Pain: Clinical Manual, St. Louis: Mosby, 1999 at p. 16). The symptoms can also be reported or measured by others, again either qualitatively or quantitatively.

Measurements of skin and facial flushing can be quantitated using a chromameter (see, for example, the studies done in Muizzuddin et al., J. Soc. Cosmet. Chem. 41:369-378 (1990) and Alaluf et al., J. Nutr. 132:399-403 (2002)). Chromameters are commercially available from vendors such as Konica Minolta (CR-400 or CR-410 Chroma Meter) or Gigahertz-Optik (HCT-99D color meter). Typically, baseline readings at one or more wavelengths are taken, and the baseline values are subtracted from subsequent readings. Flushing of the skin, especially facial flushing, is a very common symptom of the alcohol flush reaction, and can be measured non-invasively and in a time-dependent manner.

Blood (plasma or serum) concentrations of acetaldehyde can be readily measured using techniques known in the art (see, for example, McCarver-May, D. et al., Journal of Analytical Toxicology, 21:134 (1997) and Badawy et al., Alcohol and Alcoholism 46:651-660 (2011), which discusses issues in measurement of total whole blood acetaldehyde). This provides an objective measurement of acetaldehyde blood levels and can also be determined in a time-dependent manner.

### Increase of biomarkers and measurement and quantitation of biomarkers

The effects of various agents on the levels of various biomarkers, such as Coenzyme A, glutathione, and NAD, can be measured by a variety of techniques. Measurement of biomarker levels can provide an indirect measurement of the degree of protection provided by a particular dosage regimen, and/or can guide a subject in choice of, or modification of, a dosage regimen. Blood levels of biomarkers can be measured in whole blood, in plasma, in serum, or in extracts thereof.

NAD concentration can be measured by extraction from blood samples, and quantitated by enzymatic spectrophotometric methods, such as the procedure described in Majamaa et al., "Increase of Blood NAD+ and Attenuation of Lacticacidemia During Nicotinamide Treatment of a Patient with the MELAS Syndrome," Life Sciences, volume 58(8): (1996).

Coenzyme A concentrations can be measured by Cayman Coenzyme A Assay Kit f (Cayman Chemical Company, Ann Arbor, Michigan, USA; Item Number 700440). Coenzyme A concentrations can be measured in plasma, serum, urine, cell lysates, or tissue homogenates. The compositions described herein may raise measurable blood Coenzyme A concentrations which are reflective of intracellular concentrations in erythrocytes, or red blood cells (RBCs).

Glutathione concentrations can measured by High-Performance Liquid Chromatography (HPLC) after blood collection, or by colorimetric methods, such as the 5,5'-dithiobis-2-nitrobenzoic acid assay described in Johnston et al., "Vitamin C Elevates Red Blood Cell Glutathione in Healthy Adults," American Journal of Clinical Nutrition, volume 58(1):103 (1993) and in Beutler et al., "Improved method for the determination of blood glutathione," J. Lab. Clin. Med. 61:882-888 (1963).

All-trans-retinoic acid concentration can be measured by High Performance Liquid Chromatography (HPLC) after blood collection by various methods, such as those described in De Leenheer et al., "All-trans-retinoic acid: measurement of reference values in human serum by high performance liquid chromatography," Journal of Lipid Research, 23(9): 1362-1367 (1982).

The compositions described herein may raise measurable blood NAD concentration, which is reflective of the intracellular NAD concentration of erythrocytes, or red blood cells (RBCs).

The NAD concentration may be measured by extraction from blood samples, and measured by enzymatic spectrophotometric methods (Majamaa et al).

As described herein is a method of raising RBC NAD concentration, through daily supplementation to prevent or reduce the alcohol flush reaction, by at least about 20 umol/L. As described herein is a method of raising RBC NAD concentration, through daily supplementation to prevent or reduce the alcohol flush reaction, by at least about 10 umol/L. As described herein is a method of raising RBC NAD concentration, through daily supplementation to prevent or reduce the alcohol flush reaction, by at least about 4 umol/L. Increases are measured relative to the levels of NAD prior to the start of supplementation.

Also described herein is a method of raising RBC NAD concentration, through daily supplementation to prevent or reduce the alcohol flush reaction, by at least 500%. As described herein is a method of raising RBC NAD concentration, through daily supplementation to prevent or reduce the alcohol flush reaction, by at least about 300%. As described herein is a method of raising RBC NAD concentration, through daily supplementation to prevent or reduce the alcohol flush reaction, by at least about 100%. As described herein is a method of raising RBC NAD concentration, through daily supplementation to prevent or reduce the alcohol flush reaction, by at least about 50%. Increases are measured relative to the levels of NAD prior to the start of supplementation.

As described herein is a method of raising blood glutathione concentration, through daily supplementation to prevent or reduce the alcohol flush reaction, by at least about 1 mmol/L. As described herein is a method of raising blood glutathione concentration, through daily supplementation to prevent or reduce the alcohol flush reaction, by at least about 0.5 mmol/L. As described herein is a method of raising blood glutathione concentration, through daily supplementation to prevent or reduce the alcohol flush reaction, by at least about 0.25 mmol/L. As described herein is a method of raising blood glutathione concentration, through daily supplementation to prevent or reduce the alcohol flush reaction, by at least about 0.1 mmol/L. Increases are measured relative to the levels of glutathione prior to the start of supplementation.

As described herein is a method of raising blood glutathione concentration, through daily supplementation to prevent or reduce the alcohol flush reaction, by at least about 50%. As described herein is a method of raising blood glutathione concentration, through daily supplementation to prevent or reduce the alcohol flush reaction, by at least about 25%. As described herein is a method of raising blood glutathione concentration, through daily supplementation to prevent or reduce the alcohol flush reaction, by at least about 10%. As described herein is a method of raising blood glutathione concentration, through daily supplementation to prevent or reduce the alcohol flush reaction, by at least about 5%. Increases are measured relative to the levels of glutathione prior to the start of supplementation.

As described herein is a method of raising blood all-trans retinoic acid concentration, through daily supplementation to prevent or reduce the alcohol flush reaction, by at least about 2 ng/mL. As described herein is a method of raising blood all-trans retinoic acid concentration, through daily supplementation to prevent or reduce the alcohol flush reaction, by at least about 1 ng/mL. As described herein is a method of raising blood all-trans retinoic acid concentration, through daily supplementation to prevent or reduce the alcohol flush reaction, by at least about 0.5 ng/mL. As described herein is a method of raising blood all-trans retinoic acid concentration, through daily supplementation to prevent or reduce the alcohol flush reaction, by at least about 0.25 ng/mL. Increases are measured relative to the levels of all-trans retinoic acid prior to the start of supplementation.

As described herein is a method of raising blood all-trans retinoic acid concentration, through daily supplementation to prevent or reduce the alcohol flush reaction, by at least about 75%. As described herein is a method of raising blood all-trans retinoic acid concentration, through daily supplementation to prevent or reduce the alcohol flush reaction, by at least about 50%. As described herein is a method of raising blood all-trans retinoic acid concentration, through daily supplementation to prevent or reduce the alcohol flush reaction, by at least about 25%. As described herein is a method of raising blood all-trans retinoic acid concentration, through daily supplementation to prevent or reduce the alcohol flush reaction, by at least about 10%. Increases are measured relative to the levels of all-trans retinoic acid prior to the start of supplementation.

### Ingredients of the anti-alcohol flush reaction supplement

The combination anti-alcohol flush reaction supplement comprises various ingredients, which are described herein.

*Agents enhancing intracellular or intramitochondrial concentration of NAD, such as nicotinamide:* As mutant ALDH2 has a Michaelis constant K_{M} for NAD about 150 times higher than wild-type, a much higher concentration of NAD is required for optimal enzyme activity. Thus, elevation of the concentration of NAD provides more cofactor for ALDH2, increasing the breakdown of acetaldehyde at a corresponding rate, reducing the level of accumulation.

Nicotinamide (niacinamide) is a preferred agent for enhancing intracellular and intramitochondrial NAD concentrations. Nicotinamide, or Vitamin B3, is a precursor for NAD. One study indicated that nicotinamide supplementation was responsible for raising NAD concentrations 24 fold. The amount of nicotinamide in the supplement can range from about 10 mg to about 2,500 mg, from 500 mg to about 1,500 mg, preferably about 750 mg to about 1,250 mg, more preferably about 1000 mg or about 750 mg.

If niacin is used as the agent for enhancing NAD concentrations, the amount of niacin in the supplement can range from about 10 mg to 2,500 mg, or from about 500 mg to 1,500 mg. In another embodiment the amount of niacin in the supplement is about 1000 mg. In another embodiment the amount of niacin in the supplement is about 750 mg.

If nicotinamide riboside is used as the agent for enhancing NAD concentrations, the amount of nicotinamide riboside in the supplement can range from about 10 mg to 2,500 mg, or from about 500 mg to 1,500 mg. In another embodiment the amount of nicotinamide riboside in the supplement is about 1000 mg. In another embodiment the amount of nicotinamide riboside in the supplement is about 750 mg.

If oxaloacetate is used is used as the agent for enhancing NAD concentrations, the amount of oxaloacetate in the supplement can range from about 10 mg to 1000 mg, or from about 50 mg to 250 mg. In another embodiment the amount of oxaloacetate in the supplement is about 150 mg. In another embodiment the amount of oxaloacetate in the supplement is about 200 mg.

*Agents enhancing glutathione concentration, such as N-Acetyl Cysteine:* N-Acetyl Cysteine (NAC) is a preferred agent used to enhance the level of the anti-oxidant glutathione. Other agents that can be used for this purpose include Vitamin C, alpha lipoic acid, L-cysteine, glutathione, glycine, L-glutamic acid, adenosylmethionine, or conjugated linoleic acid. Cysteine is a precursor to glutathione, which can help protect against cellular damage. NAC is more bioavailable than L-cysteine itself and serves multiple purposes. Both NAC as well as glutathione contain sulfur groups that can react directly with acetaldehyde, clearing it from the cell. Glutathione is an activator of the enzyme ALDH2, increasing the breakdown of acetaldehyde, as well as an inhibitor of ADH, reducing acetaldehyde build-up. Finally, NAC is also a necessary precursor for the compound Coenzyme A. When acetaldehyde is converted to acetic acid by ALDH2, the acetic acid produced is bound to Coenzyme A and enters the Citric Acid Cycle to produce energy for the cell. Thus, enhancement of Coenzyme A levels is advantageous in clearing the acetic acid. Together with pantothenic acid, NAC can raise Coenzyme A levels and enhance the enzyme kinetics of the ALDH2 reaction. Coenzyme A is also a slight inhibitor of ADH, reducing acetaldehyde build-up.

If N-acetyl cysteine is used, the amount of N-acetyl cysteine in the supplement can range from about 10 mg to about 2,000 mg, or from about 50 mg to about 1000 mg, or from about 100 mg to about 500 mg, or from about 200mg to about 500 mg, or from about 500mg, to about 1000mg. In another embodiment the amount of N-Acetyl Cysteine in the supplement is about 500 mg. In another embodiment the amount of N-Acetyl Cysteine in the supplement is about 300 mg. In other embodiments, the amount of N-acetyl cysteine in the supplement can range from about 200 mg to about 600 mg, or about 300 mg to about 500 mg, or about 400 mg.

If Vitamin C is used, the amount of Vitamin C in the supplement can range from about 10 mg to about 3,000 mg, or from about 50 mg to about 1000 mg, or from about 100 mg to about 500 mg, or from about 200 mg to about 500 mg, or from about 500 mg to about 2,000 mg. In another embodiment the amount of Vitamin C in the supplement is about 1000 mg. In another embodiment the amount of Vitamin C in the supplement is about 150 mg. In another embodiment the amount of Vitamin C in the supplement is about 500 mg.

If alpha lipoic acid is used, the amount of alpha lipoic acid in the supplement can range from about 10 mg to about 500 mg, or from about 50 mg to about 300 mg, or from about 100 mg to about 300 mg. In another embodiment the amount of alpha lipoic acid in the supplement is about 150 mg. In another embodiment the amount of alpha lipoic acid in the supplement is about 250 mg.

If glutathione is used, the amount of glutathione in the supplement can range from about 10 mg to about 1,500 mg, or from about 100 mg to about 1000 mg, or from about 100 mg to about 500 mg. In another embodiment the amount of glutathione in the supplement is about 500 mg.

If L-cysteine is used, the amount of L-cysteine in the supplement can range from about 10 mg to about 2,000 mg, or from about 50 mg to about 1000 mg, or from about 100 mg to about 500 mg, or from about 200 mg to about 500 mg, or from about 500 mg to about 1000 mg. In another embodiment the amount of L-cysteine in the supplement is about 500 mg. In another embodiment the amount of L-cysteine in the supplement is about 300 mg.

If glycine is used, the amount of glycine in the supplement can range from about 10 mg to about 2000 mg, or from about 50 mg to about 1000 mg, or from about 100 mg to about 500 mg, or from about 200 mg to about 500 mg, or from about 500 mg to about 1000 mg. In another embodiment the amount of glycine in the supplement is about 500 mg. In another embodiment the amount of glycine in the supplement is about 300 mg.

If L-glutamic acid is used, the amount of L-glutamic acid in the supplement can range from about 10 mg to about 2,000 mg, or from about 50 mg to about 1000 mg, or from about 100 mg to about 500 mg, or from about 200 mg to about 500 mg, or from about 500 mg to about 1000 mg. In another embodiment the amount of L-glutamic acid in the supplement is about 500mg. In another embodiment the amount of L-glutamic acid in the supplement is about 300mg.

If adenosylmethionine (SAMe) is used, the amount of SAMe in the supplement can range from about 10 mg to about 500 mg, or from about 50 mg to about 250 mg. In another embodiment the amount of SAMe in the supplement is about 150 mg. In another embodiment the amount of SAMe in the supplement is about 250 mg.

If conjugated linoleic acid is used, the amount of conjugated linoleic acid in the supplement can range from about 10 mg to about 500 mg, or from about 50 mg to about 300 mg, or from about 100 mg to about 300 mg. In another embodiment the amount of conjugated linoleic acid in the supplement is about 150 mg. In another embodiment the amount of conjugated linoleic acid in the supplement is about 250 mg.

*Anti-oxidants, such as Vitamin C:* The supplement also includes at least one anti-oxidant. Vitamin C is a well-documented anti-oxidant. Supplementation with Vitamin C can increase levels of glutathione by 50% in only two weeks. In addition, Vitamin C has general protective properties as an anti-oxidant, and is relatively safe at high doses.

The amount of Vitamin C in the supplement can range from about 100 mg to about 500 mg, preferably about 200 mg to about 400 mg, more preferably about 300 mg.

Vitamin E or other anti-oxidants can be employed instead of or in addition to Vitamin C. Alternatively, alpha-lipoic acid, another agent used in the supplement as a separate ingredient, can also be employed as an anti-oxidant.

*Alpha Lipoic Acid:* Alpha Lipoic Acid is an anti-oxidant, and its use in the supplement helps to maintain high concentrations of glutathione. This occurs due to regeneration of reduced glutathione. The R-enantiomer of alpha lipoic acid is used in the supplement (5-[(3R)-dithiolan-3-yl]pentanoic acid).

The amount of alpha lipoic acid in the supplement can range from about 50 mg to about 250 mg, preferably about 100 mg to about 200 mg, more preferably about 150 mg. If alpha lipoic acid is also used as the anti-oxidant, approximately double this amount is used, that is, from about 100 mg to about 500 mg, preferably about 200 mg to about 400 mg, more preferably about 300 mg. If racemic alpha lipoic acid is used in the supplement, the foregoing amounts are doubled, so as to provide the specified amount of the R-isomer in the supplement. That is, if 300 mg of R-alpha lipoic acid is desired in the supplement, and the racemic mixture is used to make the supplement, 600 mg of the racemic mixture is used in order to provide 300 mg of the R-enantiomer.

*Agents enhancing the level of Coenzyme A, such as pantothenic acid:* As described previously, increased Coenzyme A has two functions in lowering blood acetaldehyde. Pantothenic acid is a precursor to Coenzyme A and is a preferred agent for use in the supplement to enhance the levels of Coenzyme A.

The agent capable of raising intracellular Coenzyme A glutathione concentration in the subject can be pantothenic acid, pantethine, pantetheine, 4'-phosphopantothenate, 4'-phosphopantetheine, N-acetyl-cysteine, or L-cysteine.

If pantothenic acid is used (also known as pantothenate), the amount of pantothenic acid in the supplement can range from about 10 mg to 1000 mg, or from about 100 mg to 500 mg, or from about 50 mg to about 250 mg, or from about 100 mg to about 200 mg, or about 150 mg. In another embodiment the amount of pantothenic acid in the supplement is about 50 mg. In another embodiment the amount of pantothenic acid in the supplement is about 250 mg.

If pantethine is used, the amount of pantethine in the supplement can range from about 10 mg to about 1000 mg, or from about 100 mg to about 500 mg. In another embodiment the amount of pantethine in the supplement is about 50 mg. In another embodiment the amount of pantethine in the supplement is about 250 mg.

If 4'-phosphopantetheine is used, the amount of 4'-phosphopantetheine in the supplement can range from about 10 mg to about 1000 mg, or from about 100 mg to about 500 mg. In another embodiment the amount of 4'-phosphopantetheine in the supplement is about 50 mg. In another embodiment the amount of 4'-phosphopantetheine in the supplement is about 250 mg.

If 4'-phosphopantothenate is used, the amount of 4'-phosphopantothenate in the supplement can range from about 10 mg to about 1000 mg, or from about 100 mg to about 500 mg. In another embodiment the amount of 4'-phosphopantothenate in the supplement is about 50 mg. In another embodiment the amount of 4'-phosphopantothenate in the supplement is about 250 mg.

If N-acetyl cysteine is used, the amount of N-acetyl cysteine in the supplement can range from about 10mg to about 2,000 mg, or from about 50 mg to about 1000 mg, or from about 100 mg to about 500 mg, or from about 200 mg to about 500 mg, or from about 500 mg to about 1000 mg. In another embodiment the amount of N-acetyl cysteine in the supplement is about 500 mg. In another embodiment the amount of N-acetyl cysteine in the supplement is about 300 mg.

If L-cysteine is used, the amount of L-cysteine in the supplement can range from about 10 mg to about 2,000 mg, or from about 50 mg to about 1000 mg, or from about 100 mg to about 500 mg, or from about 200 mg to about 500 mg, or from about 500 mg to about 1000 mg. In another embodiment the amount of L-cysteine in the supplement is about 500 mg. In another embodiment the amount of L-cysteine in the supplement is about 300 mg.

*Agents enhancing the level of All-Trans Retinoic Acid:* beta-carotene or a retinol ester can be used to raise concentrations of all-trans retinoic acid in a subject. Examples of esters of retinol that can be used are retinyl arachidonate, retinyl stearate, retinyl oleate, retinyl linoleate, retinyl heptadecanoate, retinyl palmitate, retinyl myristate, retinyl laurate, retinyl caprylate, or retinyl acetate.

If beta-carotene is used, the amount of beta-carotene in the supplement can range from about 100 IU to about 15,000 IU, or from about 1000 IU to about 10,000 IU. In another embodiment the amount of beta-carotene in the supplement is about 2.500IU. In another embodiment the amount of beta-carotene in the supplement is about 5000 IU.

If a retinol ester is used, the amount of retinol ester in the supplement can range from about 100 IU to about 15,000 IU, or from about 1000 IU to about 10,000 IU. In another embodiment the amount of retinol ester in the supplement is about 2500 IU. In another embodiment the amount of retinol ester in the supplement is about 5000 IU.

*Silybum marianum extract. Silybum marianum* (milk thistle) is an annual or biannual plant of the Asteraceae family. It has been used as a medicinal plant for centuries, and its extract is used in medicine under the name silymarin. Milk thistle is typically extracted with 95% ethanol, which may then be filtered and dried by solvent evaporation. The resulting extract typically contains 1.5-3% flavonolignans (referred to collectively as silymarin; the principal components are silybin and isosilybin), 20-30% fatty acids (including linoleic, oleic, and palmitic acids), 25-30% proteins, and small amounts of vitamin E and various sterols. It may be included in the any of the compositions as disclosed herein at concentrations between 100-5000 mg/day. Additional information regarding this extract is available from the World Health Organization at URL apps.who.int/medicinedocs/pdf/s4927e/s4927e.pdf, pages 300-316.

*Hovenia dulcis fruit extract. Hovenia dulcis* (oriental raisin tree) is a tree found in Asia, over Eastern China, Korea, and in the Himalayan mountains (found at altitudes up to 2,000 m). There is evidence that extracts of the *Hovenia dulcis* fruit are hepatoprotective and can reduce blood alcohol levels (see Hyun et al., Planta Medica 76:943-949 (2010) and references therein). The main chemical for this effect in *Hovenia dulcis* is quercetin, which is believed to act as an anti-inflammatory and anti-oxidant. Another component of *Hovenia dulcis*, ampelopsin (dihydromyricetin), also affects alcohol metabolism and is discussed in Shen et al., The Journal of Neuroscience, 32(1): 390-401 (2012). For treating a human patient, a typical daily dose of the above-mentioned extract may range from about 0.01 to 10 g/kg body weight, preferably 1 to 5 g/kg body weight. A lower alcohol-insoluble fraction and the polysaccharide isolated therefrom can be used in tablet or capsule form, or may be added to food or beverage. The amount of the fraction and/or polysaccharide may generally range from about 0.1 to 15 w/w %, preferably 1 to 10 w/w % based on the total weight of food, and 0.1 to 15 g, preferably 1 to 10 g.

### Dosing/administration

The compositions described herein comprising the supplement can be administered in various regimens. The compositions can be administered to a subject prior to consumption of alcohol by the subject. The compositions can be administered to the subject for at least three days prior to consumption of alcohol by the subject, such as periods of at least about seven days, at least about 14 days, at least about a month, or at least about three months.

The compositions can be administered in a single daily dose, where the dose contains the entire amount of the composition to be administered on that day. Alternatively, the compositions can be administered in multiple divided doses over the course of one day, such that the multiple doses administered add up to the entire amount of the composition to be administered on that day. Thus, one-half of the daily dosage can be administered twice a day, one-third of the daily dosage can be administered three times a day, one-fourth of the daily dosage can be administered four times a day, one-fifth of the daily dosage can be administered five times a day, etcetera. The individual components of the supplement may be administered separately, or in any sub-combination, over the course of the day; for example, the amount of anti-oxidant and the amount of alpha-lipoic acid can be administered in the morning, and the amount of the agents capable of increasing intracellular or intramitochondrial concentration of NAD, of increasing glutathione concentration, and of increasing Coenzyme A can be administered in the evening.

### Formulations and Routes of Administration

For the purposes of this disclosure, the compounds may be administered by a variety of means including orally, parenterally, by inhalation spray, topically, or rectally in formulations containing pharmaceutically acceptable carriers, adjuvants and vehicles. The term oral as used herein includes, but is not limited to sublingual and buccal. Oral administration includes fluid drinks, energy bars, as well as pill formulations. Oral administration is advantageous due to its convenience. However, a patient who has consumed a great deal of alcohol may have difficulty in swallowing, or may be unconscious, in which case introduction of the supplements by other means (feeding tube, intravenously, etc.) may be desirable.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents; such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Formulations for oral use may be also presented as hard gelatin capsules where the active ingredient is mixed with an inert solid diluent, for example calcium phosphate or kaolin, or as soft gelatin capsules wherein the active ingredient is mixed with water or an oil medium, such as peanut oil, liquid paraffin or olive oil.

Aqueous suspensions of the disclosure contain the active materials in admixture with excipients suitable for the manufacture of aqueous-suspensions. Such excipients include a suspending agent, such as sodium carboxymethylcellulose, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension may also contain one or more preservatives such as ethyl or n-propyl p-hydroxy-benzoate, one or more coloring agents, one or more flavoring agents and one or more sweetening agents, such as sucrose or saccharin.

Oil suspensions may be formulated by suspending the active ingredient in a vegetable oil, such as arachis oil, olive oil, sesame oil or coconut oil, or a mineral oil such as liquid paraffin. The oral suspensions may contain a thickening agent, such as beeswax, hard paraffin or cetyl alcohol. Sweetening agents, such as those set forth above, and flavoring agents may be added to provide a palatable oral preparation. These compositions may be preserved by the addition of an anti-oxidant such as ascorbic acid.

Dispersible powders and granules of the disclosure suitable for preparation of an aqueous suspension by the addition of water provide the active ingredient in admixture with a dispersing or wetting agent, a suspending agent, and one or more preservatives. Suitable dispersing or wetting agents and suspending agents are exemplified by those disclosed above. Additional excipients, for example sweetening, flavoring and coloring agents, may also be present.

The pharmaceutical compositions of the disclosure may also be in the form of oil-in-water emulsions. The oily phase may be a vegetable oil, such as olive oil or arachis oil, a mineral oil, such as liquid paraffin, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan monooleate, and condensation products of these partial esters with ethylene oxide, such as polyoxyethylene sorbitan monooleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

As noted above, formulations of the disclosure suitable for oral administration may be presented as discrete units such as capsules, cachets or tablets each containing a predetermined amount of the active ingredient, as a powder or granules; as a solution or a suspension in an aqueous or non-aqueous liquid, or as an oil-in-water liquid emulsion or a water-in-oil liquid emulsion. The active ingredient may also be administered as a bolus, electuary or paste.

A tablet may be made by compression or molding, optionally with one or more accessory ingredients. Compressed tablets may be prepared by compressing in a suitable machine the active ingredient in a free flowing form such as a powder or granules, optionally mixed with a binder (e.g., povidone, gelatin, hydroxypropyl ethyl cellulose), lubricant, inert diluent, preservative, disintegrant (e.g., sodium starch glycolate, cross-linked povidone, cross-linked sodium carboxymethyl cellulose) surface active or dispersing agent. Molded tablets may be made by molding in a suitable machine a mixture of the powdered compound moistened with an inert liquid diluent. The tablets may optionally be coated or scored and may be formulated so as to provide. slow or controlled release of the active ingredient therein using, for example, hydroxypropyl methylcellulose in varying proportions to provide the desired release profile. Tablets may optionally be provided with an enteric coating, to provide release in parts of the gut other than the stomach. This is particularly advantageous with the compounds of formula 1 when such compounds are susceptible to acid hydrolysis.

Formulations suitable for topical administration in the mouth include lozenges comprising the active ingredient in a flavored base, usually sucrose and acacia or tragacanth; pastilles comprising the active ingredient in an inert base such as gelatin and glycerin, or sucrose and acacia; and mouthwashes comprising the active ingredient in a suitable liquid carrier.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

As used herein, pharmaceutically acceptable salts include, but are not limited to: acetate, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydoxymethyl)aminomethane. Additional pharmaceutically acceptable salts are known to those skilled in the art.

The compositions described herein may also be formulated as nutraceutical compositions. The term "nutraceutical composition" as used herein refers to a food product, foodstuff, dietary supplement, nutritional supplement or a supplement composition for a food product or a foodstuff comprising the compositions described herein added exogenously. As used herein, the term food product refers to any food or feed suitable for consumption by humans or animals. The food product may be a prepared and packaged food (e.g., mayonnaise, salad dressing, bread, grain bar, beverage, etc.) or an animal feed (e.g., extruded and pelleted animal feed, coarse mixed feed or pet food composition). As used herein, the term foodstuff refers to any substance fit for human or animal consumption.

Food products or foodstuffs are for example beverages such as non-alcoholic drinks as well as liquid preparation to be added to drinking water and liquid food, non-alcoholic drinks are for instance soft drinks, sport drinks, fruit juices, such as for example orange juice, apple juice and grapefruit juice; lemonades, teas, near-water drinks and milk and other dairy drinks such as for example yoghurt drinks, and diet drinks. In another embodiment food products or foodstuffs refer to solid or semi-solid foods comprising the composition describd herein. These forms can include, but are not limited to baked goods such as cakes and cookies, puddings, dairy products, confections, snack foods, or frozen confections or novelties (e.g., ice cream, milk shakes), prepared frozen meals, candy, snack products (e.g., chips), liquid food such as soups, spreads, sauces, salad dressings, prepared meat products, cheese, yogurt and any other fat or oil containing foods, and food ingredients (e.g., wheat flour).

Animal feed including pet food compositions advantageously include food intended to supply necessary dietary requirements, as well as treats (e.g., dog biscuits) or other food supplements. The animal feed comprising the composition described herein may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (e.g., biscuits) or any other delivery form.

The term dietary supplement refers to a small amount of a compound for supplementation of a human or animal diet packaged in single or multiple dose units. Dietary supplements do not generally provide significant amounts of calories but may contain other micronutrients (e.g., vitamins or minerals). The term food products or foodstuffs also includes functional foods and prepared food products pre-packaged for human consumption.

The term nutritional supplement refers to a composition comprising a dietary supplement in combination with a source of calories. In some embodiments, nutritional supplements are meal replacements or supplements (e.g., nutrient or energy bars or nutrient beverages or concentrates).

Dietary supplements described herein may be delivered in any suitable format. In preferred embodiments, dietary supplements are formulated for oral delivery. The ingredients of the dietary supplement described herein are contained in acceptable excipients and/or carriers for oral consumption. The actual form of the carrier, and thus, the dietary supplement itself, is not critical. The carrier may be a liquid, gel, gelcap, capsule, powder, solid tablet (coated or non- coated), tea, or the like. The dietary supplement is preferably in the form of a tablet or capsule and most preferably in the form of a hard (shell) capsule. Suitable excipient and/or carriers include maltodextrin, calcium carbonate, dicalcium phosphate, tricalcium phosphate, microcrystalline cellulose, dextrose, rice flour, magnesium stearate, stearic acid, croscarmellose sodium, sodium starch glycolate, crospovidone, sucrose, vegetable gums, lactose, methylcellulose, povidone, carboxymethylcellulose, corn starch, and the like (including mixtures thereof). Preferred carriers include calcium carbonate, magnesium stearate, maltodextrin, and mixtures thereof. The various ingredients and the excipient and/or carrier are mixed and formed into the desired form using conventional techniques. The tablet or capsule described herein may be coated with an enteric coating that dissolves at a pH of about 6.0 to 7.0. A suitable enteric coating that dissolves in the small intestine but not in the stomach is cellulose acetate phthalate.

In other embodiments, the dietary supplement is provided as a powder or liquid suitable for adding by the consumer to a food or beverage. For example, in some embodiments, the dietary supplement can be administered to an individual in the form of a powder, for instance to be used by mixing into a beverage, or by stirring into a semi-solid food such as a pudding, topping, sauce, puree, cooked cereal, or salad dressing, for instance, or by otherwise adding to a food or the dietary supplement e.g. enclosed in caps of food or beverage container for release immediately before consumption. The dietary supplement may comprise one or more inert ingredients, especially if it is desirable to limit the number of calories added to the diet by the dietary supplement. For example, the dietary supplement described herein may also contain optional ingredients including, for example, herbs, vitamins, minerals, enhancers, colorants, sweeteners, flavorants, inert ingredients, and the like.

In some embodiments, the dietary supplements further comprise additional vitamins and minerals including, but not limited to, calcium phosphate or acetate, tribasic; potassium phosphate, dibasic; magnesium sulfate or oxide; salt (sodium chloride); potassium chloride or acetate; ascorbic acid; ferric orthophosphate; zinc sulfate or oxide; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3; cyanocobalamin; sodium selenite; copper sulfate; vitamin A; vitamin C; inositol; potassium iodide. Suitable dosages for vitamins and minerals may be obtained, for example, by consulting the U.S. RDA guidelines and the disclosure herein.

As described herein are nutritional supplements (e.g., energy bars or meal replacement bars or beverages) comprising the composition as described herein. The nutritional supplement may serve as meal or snack replacement and generally provide nutrient calories. Preferably, the nutritional supplements provide carbohydrates, proteins, and fats in balanced amounts. The nutritional supplement can further comprise carbohydrate, simple, medium chain length, or polysaccharides, or a combination thereof. A simple sugar can be chosen for desirable organoleptic properties. Uncooked cornstarch is one example of a complex carbohydrate. If it is desired that it should maintain its high molecular weight structure, it should be included only in food formulations or portions thereof which are not cooked or heat processed since the heat will break down the complex carbohydrate into simple carbohydrates, wherein simple carbohydrates are mono- or disaccharides. The nutritional supplement contains, in one embodiment, combinations of sources of carbohydrate of three levels of chain length (simple, medium and complex; e.g., sucrose, maltodextrins, and uncooked cornstarch).

Sources of protein to be incorporated into the nutritional supplement described herein can be any suitable protein utilized in nutritional formulations and can include whey protein, whey protein concentrate, whey powder, egg, soy flour, soy milk soy protein, soy protein isolate, caseinate (e.g., sodium caseinate, sodium calcium caseinate, calcium caseinate, potassium caseinate), animal and vegetable protein and hydrolysates or mixtures thereof. When choosing a protein source, the biological value of the protein should be considered first, with the highest biological values being found in caseinate, whey, lactalbumin, egg albumin and whole egg proteins. In a preferred embodiment, the protein is a combination of whey protein concentrate and calcium caseinate. These proteins have high biological value; that is, they have a high proportion of the essential amino acids. See Modern Nutrition in Health and Disease, 8th ed., Lea & Febiger, 1986, especially Volume 1, pages 30-32. The nutritional supplement can also contain other ingredients, such as one or a combination of other vitamins, minerals, anti-oxidants, fiber and other dietary supplements (e.g., protein, amino acids, choline, lecithin). Selection of one or several of these ingredients is a matter of formulation, design, consumer preferences and end- user. The amounts of these ingredients added to the dietary supplements described herein are readily known to the skilled artisan. Guidance to such amounts can be provided by the U.S. RDA doses for children and adults. Further vitamins and minerals that can be added include, but are not limited to, calcium phosphate or acetate, tribasic; potassium phosphate, dibasic; magnesium sulfate or oxide; salt (sodium chloride); potassium chloride or acetate; ascorbic acid; ferric orthophosphate; niacinamide; zinc sulfate or oxide; calcium pantothenate; copper gluconate; riboflavin; beta-carotene; pyridoxine hydrochloride; thiamin mononitrate; folic acid; biotin; chromium chloride or picolonate; potassium iodide; sodium selenate; sodium molybdate; phylloquinone; vitamin D3 ; cyanocobalamin; sodium selenite; copper sulfate; vitamin A; vitamin C; inositol; potassium iodide.

The nutritional supplement can be provided in a variety of forms, and by a variety of production methods. For example, to manufacture a food bar, the liquid ingredients are cooked; the dry ingredients are added with the liquid ingredients in a mixer and mixed until the dough phase is reached; the dough is put into an extruder, and extruded; the extruded dough is cut into appropriate lengths; and the product is cooled. The bars may contain other nutrients and fillers to enhance taste, in addition to the ingredients specifically listed herein.

It is understood by those of skill in the art that other ingredients can be added to those described herein, for example, fillers, emulsifiers, preservatives, etc. for the processing or manufacture of a nutritional supplement.

Additionally, flavors, coloring agents, spices, nuts and the like may be incorporated into the nutraceutical composition. Flavorings can be in the form of flavored extracts, volatile oils, chocolate flavorings, peanut butter flavoring, cookie crumbs, crisp rice, vanilla or any commercially available flavoring. Examples of useful flavoring include, but are not limited to, pure anise extract, imitation banana extract, imitation cherry extract, chocolate extract, pure lemon extract, pure orange extract, pure peppermint extract, imitation pineapple extract, imitation rum extract, imitation strawberry extract, or pure vanilla extract; or volatile oils, such as balm oil, bay oil, bergamot oil, cedarwood oil, walnut oil, cherry oil, cinnamon oil, clove oil, or peppermint oil; peanut butter, chocolate flavoring, vanilla cookie crumb, butterscotch or toffee. In one embodiment, the dietary supplement contains cocoa or chocolate.

Emulsifiers may be added for stability of the nutraceutical compositions. Examples of suitable emulsifiers include, but are not limited to, lecithin (e.g., from egg or soy), and/or mono- and di- glycerides. Other emulsifiers are readily apparent to the skilled artisan and selection of suitable emulsifier(s) will depend, in part, upon the formulation and final product. Preservatives may also be added to the nutritional supplement to extend product shelf life. Preferably, preservatives such as potassium sorbate, sodium sorbate, potassium benzoate, sodium benzoate or calcium disodium EDTA are used.

In addition to the carbohydrates described above, the nutraceutical composition can contain natural or artificial (preferably low calorie) sweeteners, e.g., saccharides, cyclamates, aspartamine, aspartame, acesulfame K, and/or sorbitol. Such artificial sweeteners can be desirable if the nutritional supplement is intended to be consumed by an overweight or obese individual, or an individual with type II diabetes who is prone to hyperglycemia.

In addition to the preparations described above, standard pharmaceutical preparations, such as those described in Remington: The Science and Practice of Pharmacy (22nd Edition, Pharmaceutical Press, 2012 and 21st Edition, Pharmaceutical Press, 2011), can be used.

### Kits

Also described herein are articles of manufacture and kits containing the compositions described herein. The article of manufacture comprises a container with a label. Suitable containers include, for example, bottles, vials, and test tubes. The containers may be formed from a variety of materials such as glass or plastic. The containers can hold one or more of the individual ingredients. The label on the container indicates that the kit is used for prevention or reduction of one or more symptoms associated with the alcohol flush reaction.

### EXAMPLES

### Example 1

### Supplement Formulations

The supplement can be prepared with the following amounts of individual components:
*Formulation A:*
   Nicotinamide: 1000 mg
   N Acetyl Cysteine: 400 mg
   Vitamin C: 300 mg
   Alpha-Lipoic Acid: 150 mg
   Pantothenic Acid: 150 mg
*Formulation B:*
   Nicotinamide: 800 mg
   N Acetyl Cysteine: 300 mg
   Vitamin C: 150 mg
   Alpha-Lipoic Acid: 100 mg
   Pantothenic Acid: 100 mg
*Formulation C:*
   Nicotinamide: 700 mg
   N Acetyl Cysteine: 300 mg
   Vitamin C: 150 mg
   Pantothenic Acid: 100 mg
   Vitamin A: 2500 IU
   Thiamine: 50mg
*Formulation D:*
   N Acetyl Cysteine: 300 mg
   Nicotinamide: 700 mg
   Pantothenic Acid: 100 mg

### Reference Example 2

### Demonstration of Efficacy of Combination Anti-Alcohol Flush Reaction Supplement Therapy

The efficacy of the combination anti-alcohol flush reaction supplement (hereafter "supplement") is demonstrated in the following manner. A double blind supplement trial is conducted where subjects take the supplement (or placebo) once daily for three weeks. Either Formulation A or Formulation B of Example 1 is used with all subjects. Subjects provide informed consent for participation in accordance with all laws and regulations, and all policies of the institution where the study is conducted. The age of subjects is verified to ensure that they are of legal age to consume alcohol in the jurisdiction where the study takes place (typically, age 21 years or older in the United States). Subjects that qualify under the inclusion criteria of the study come in for a first visit, where their health and ability to participate in the study are assessed by the staff. At this initial visit, subjects first have their blood acetaldehyde level measured before drinking any alcohol. Subjects then consume two standard alcoholic drinks. Three more blood tests for acetaldehyde after alcoholic beverage consumption are conducted at time points of 15 minutes, 45 minutes and 90 minutes after finishing the drinks. Each patient must have suitable transportation to and from the research facility in order to avoid driving while under the influence of alcohol. Patient privacy and confidentiality is maintained according to law and regulation. Patients can also fill out questionnaires indicating the degree to which they experience facial flushing, dizziness, nausea, headache, stupor, or increased heart rate at the same time points at which the blood tests are conducted. The degree of facial flushing can also be reported by the staff (either by visual observation or by measurement with a chromameter), and heart rate can be monitored.

Subjects commence taking supplement (or placebo) daily for three weeks, starting the day after the initial visit. The double-blind trial utilizes three groups: one group consisting of individuals with ALDH2 deficiency who take the supplement, one group of individuals without the deficiency (that is, they do not demonstrate the alcohol flush reaction) who take the supplement, and a third group of individuals who do have ALDH2 deficiency, but who take a placebo.

The subjects visit the research facility again three weeks later. The same steps are performed as were conducted during the initial visit of the subjects. Again, subjects have a blood test to determine acetaldehyde levels, consume two alcoholic drinks, and have three more blood tests at time points of 15 minutes, 45 minutes and 90 minutes after finishing the drinks in order to measure the levels of acetaldehyde in the blood. Patients can also fill out questionnaires indicating the degree to which they experience facial flushing, dizziness, nausea, or increased heart rate at the same time points at which the blood tests are conducted. The degree of facial flushing can also be reported by the staff (using the same method-either by visual observation or by measurement with a chromameter-as used previously), and heart rate can be monitored. The data is compared between visits one and two to demonstrate the effects of the supplement in preventing accumulation of acetaldehyde.

### Example 3

### Demonstration of Efficacy of Combination Anti-Alcohol Flush Reaction Supplement Therapy

The efficacy of the combination anti-alcohol flush reaction supplement (hereafter "supplement") is demonstrated in the following manner. A double blind supplement trial is conducted where subjects take the supplement (or placebo) once daily for 30 days. One group will not supplement for 30 days and will take a single dose prior to consuming alcohol. Either Formulation C or Formulation D of Example 1 is used with all subjects. Subjects provide informed consent for participation in accordance with all laws and regulations, and all policies of the institution where the study is conducted. The age of subjects is verified to ensure that they are of legal age to consume alcohol in the jurisdiction where the study takes place (typically, age 21 years or older in the United States). Subjects that qualify under the inclusion criteria of the study come in for a first visit, where their health and ability to participate in the study are assessed by the staff. At this initial visit there will be samples taken for a PCR genotyping to confirm that all subjects with self-reported facial flushing when drinking, are heterozygous for the ALDH2 mutation. If they do possess this mutation and pass the inclusion criteria, they will visit the facility for a 2^{nd} visit. At this 2^{nd} visit, subjects first have their blood acetaldehyde level measured before drinking any alcohol. Subjects then consume 0.3g/Kg body weight of ethanol. Three more blood tests for acetaldehyde after ethanol consumption are conducted immediately after drinking, and at two more time points, each with 20 minutes of separation. At each of these time points there will be measurements for heart rate and skin temperature. Each patient must have suitable transportation to and from the research facility in order to avoid driving while under the influence of alcohol. Patient privacy and confidentiality is maintained according to law and regulation. Patients can also fill out questionnaires indicating the degree to which they experience facial flushing, dizziness, nausea, headache, stupor, or increased heart rate at the same time points at which the blood tests are conducted. The degree of facial flushing can also be reported by the staff (either by visual observation or by measurement with a chromameter), as well as self-reported by the subject.

Subjects commence taking supplement (or placebo) daily for 30 days, starting the day after the initial visit. The double-blind trial utilizes 4 groups: one group consisting of individuals with ALDH2 deficiency who take the supplement for 30 days (formulation C), one group of individuals with the ALDH2 deficiency who take a subset of the supplement for 30 days (formulation D), one group of individuals with the ALDH2 deficiency, but who take a placebo for 30 days, and one group of individuals with the ALDH2 deficiency who do NOT supplement for 30 days, but take a single dose of the supplement (formulation C) at the third visit.

The subjects visit the research facility again for a 3^{rd} visit 30 days later. The same steps are performed as were conducted during the 2^{nd} visit, with one addition. Again, subjects have a blood test to determine acetaldehyde levels. All subjects (except placebo) will then consume one dosage, 20 minutes prior to consuming alcohol. Subjects will then consume 0.3g/Kg body weight of ethanol. Three more blood tests for acetaldehyde after ethanol consumption are conducted immediately after drinking, and at two more time points, each with 20 minutes of separation. At each of these time points there will be measurements for heart rate and skin temperature. Patients can also fill out questionnaires indicating the degree to which they experience facial flushing, dizziness, nausea, or increased heart rate at the same time points at which the blood tests are conducted. The degree of facial flushing can also be reported by the staff (using the same method-either by visual observation or by measurement with a chromameter-as used previously), and can also be self-reported by the subject. The data is compared between visits two and three to demonstrate the effects of the supplement in preventing accumulation of acetaldehyde, relevant to the placebo group.

### Reference Example 4

### Additional Demonstration of Efficacy of Combination Anti-Alcohol Flush Reaction Supplement Therapy

The efficacy of the combination anti-alcohol flush reaction supplement (hereafter "supplement") can also be demonstrated in a double blind trial, where subjects take the supplement (or placebo) twice daily for three weeks. Formulation A, Formulation B, Formulation C, or Formulation D of Example 1 is used with all subjects. Other than the twice-daily administration of supplement (or placebo), the trial is carried out as described in Reference Example 2.

### Reference Example 5

### Studies in Individuals of Efficacy of Combination Anti-Alcohol Flush Reaction Supplement Therapy

In order to evaluate the efficacy of certain supplements, as described herein, subjects took various forms of the supplements daily for two weeks. The subjects then consumed two standard alcohol beverages and their symptoms were evaluated. One hour prior to drinking the alcohol beverages, the subjects also consumed another dose of the formulation. Overt physical symptoms and self-reported physical symptoms were used as assessments for the alcohol flush reaction. These experimental tests include eleven subjects. All experimental subjects were unpaid volunteers who experience all symptoms of the alcohol flush reaction.

The following symptoms were assessed: facial redness, heart rate, self-evaluated body temperature, headache, nausea, and general physical discomfort. These are symptoms of the alcohol flush reaction that have all been directly linked with an increase in blood acetaldehyde.

Subject 1 (male) took a supplement containing 500 mg of N-Acetyl Cysteine, 500 mg Vitamin C and 1000 mg nicotinamide. After the supplementation period, upon consumption of alcohol, subject 1 experienced immediately noticeable reduction in facial redness, headache, and body temperature from what the subject typically previously experienced in response to the same amount of alcohol consumption.

Subject 2 (female) and Subject 3 (male) took a supplement containing 500 mg of N-Acetyl Cysteine, 100 mg pantothenic acid, 500 mg Vitamin C, and 1000 mg nicotinamide. Upon consumption of alcohol, Subject 2 displayed a significant reduction in both physical discomfort and headache. Subject 3 displayed mild reductions in all assessments for the alcohol flush reaction.

Subject 4 (male) took a supplement containing only with 1500 mg nicotinamide. The subject reported reductions in facial redness and heart rate.

Subject 5 (female), Subject 6 (female), and Subject 7 (male) took a supplement containing 500 mg N-Acetyl Cysteine, 100 mg Pantothenic acid, 500 mg Vitamin C, 1000 mg nicotinamide and 150 mg Alpha Lipoic Acid. Subject 5 displayed mild reductions in all alcohol flush reaction assessments, with a significant reduction in heart rate. Subject 6 displayed a significant reduction in heart rate and headache, and a reduction in physical discomfort and facial flushing. Subject 7 displayed dramatic decreases in heart rate, nausea, physical discomfort, and body temperature, and a mild reduction in facial redness.

Subject 8 (female) took a supplement containing 2500 IU Vitamin A, 700 mg Nicotinamide, 25 mg pantothenic acid, 150 mg Vitamin C, 125 mg Alpha Lipoic Acid, 300 mg N-Acetyl Cysteine, and 10 mg Thiamine. Subject 8 displayed dramatic reductions in all assessments for the alcohol flush reaction, with zero displayed facial redness.

Subject 9 (female), Subject 10 (female), and Subject 11 (male) took a supplement containing 2500 IU Vitamin A, 700 mg Nicotinamide, 50 mg Pantothenic acid, 150 mg Vitamin C, 80 mg Alpha Lipoic Acid, 300 mg N-Acetyl Cysteine, and 50 mg Thiamine. Subject 11 did not display reductions in any assessments. Subject 9 displayed a dramatic reduction in heart rate, and mild reductions in facial redness, body temperature, headache, nausea, and physical discomfort. Subject 10 displayed significant reductions in all assessments.

Different individuals display varying degrees of severity for the symptoms of the alcohol flush reaction. This suggests that at a given concentration of acetaldehyde in the blood, individuals experience varying intensities of the alcohol flush reaction symptoms assessed in these experiments. This is confirmed by the experimental results, in that 10 out of 11 individuals displayed reductions in at least one symptom, although the symptoms varied. The results suggest that various supplements, as described herein, reduce acetaldehyde in the blood, and individuals respond differently to that reduction. The most consistently reduced symptom was heart rate.

Also described herein are:
(1). A composition for use by a subject to prevent or reduce the alcohol flush reaction, said composition comprising:
   a) an agent capable of increasing intracellular or intramitochondrial concentration of NAD in the subject;
   b) an agent capable of increasing glutathione concentration in the subject;
   c) an agent which is an anti-oxidant;
   d) an agent which is alpha-lipoic acid or a precursor to alpha-lipoic acid; and
   e) an agent capable of increasing the level of Coenzyme A in the subject;
   wherein said agents are present in the composition in an amount effective to prevent or reduce the alcohol flush reaction in the subject.
(2). The composition of (1), wherein the agent capable of increasing intracellular or intramitochondrial concentration of NAD in the subject is nicotinamide.
(3). The composition of (2), wherein the amount of nicotinamide in the dosage is between about 500 mg to about 1,500 mg.
(4). The composition of any of (1)-(3), wherein the agent capable of increasing glutathione concentration in the subject is N-acetyl cysteine.
(5). The composition of (4), wherein the amount of N-acetyl cysteine in the dosage is between about 200 mg to about 600 mg.
(6). The composition of any of (1)-(5), wherein the anti-oxidant is Vitamin C.
(7). The composition of (6), wherein the amount of Vitamin C in the dosage is between about 100 mg to about 500 mg.
(8). The composition of any of (1)-(7), wherein the amount of alpha lipoic acid in the dosage is between about 50 mg to about 250 mg.
(9). The composition of any of (1)-(8), wherein the agent capable of increasing the level of Coenzyme A in the subject is pantothenic acid.
(10). The composition of (9), wherein the amount of pantothenic acid in the dosage is about 50 mg to about 250 mg.
(11). The composition of any of (1)-(9), further comprising an agent capable of raising the blood concentration of all-trans retinoic acid in a subject.
(12). A method of preventing or reducing at least one symptom of the alcohol flush reaction in a subject, comprising the step of administering to the subject a dosage of a composition according to any one of (1)-(11), or use of any composition according to (1)-(11) for preventing or reducing at least one symptom of the alcohol flush reaction in a subject.
(13). The method or use of (12), wherein the dosage is administered to the subject daily, either in a single dose per day, or in multiple divided doses per day.
(14). The method or use of (12) or (13), wherein the composition is administered to the subject for at least seven days prior to consumption of alcohol by the subject.
(15). The method or use of any of (12)-(14), wherein the at least one symptom is selected from the group consisting of facial flushing, hangover, dizziness, nausea, increased blood acetaldehyde concentration, and increased heart rate.
(16). The method or use of any of (12)-(15), wherein the at least one symptom is facial flushing.
(17). The method or use of any of (12)-(15), wherein the at least one symptom is hangover.
(18). The method or use of any of (12)-(15), wherein the at least one symptom is increased blood acetaldehyde concentration.
(19). The method or use of any of (12)-(15), wherein the at least one symptom is increased heart rate.
(20). The method or use of any of (12)-(15), wherein the at least one symptom is nausea.
(21). The method or use of any of (12)-(15), wherein the at least one symptom is dizziness.
(22). The composition of any of (1)-(11) for use in preventing or reducing at least one symptom of the alcohol flush reaction in a subject.
(23). Use of a composition of any of (1)-(11) for the manufacture of a supplement or medicament for use in preventing or reducing at least one symptom of the alcohol flush reaction in a subject.
(24). A composition comprising:
   a) an agent capable of increasing intracellular or intramitochondrial concentration of NAD in a subject; and
   b) an agent capable of increasing glutathione concentration in the subject.
(25). The composition of (24), wherein said agents are present in the composition in an amount effective to prevent or reduce the alcohol flush reaction in the subject.
(26). The composition of (24) or (25), further comprising at least one additional agent selected from the group comprising: an agent which is an anti-oxidant; an agent which is alpha-lipoic acid or a precursor to alpha-lipoic acid; and an agent capable of increasing the level of Coenzyme A in the subject.
(27). A composition comprising:
   a) an agent capable of increasing intracellular or intramitochondrial concentration of NAD in a subject;
   b) an agent capable of increasing glutathione concentration in the subject; and
   c) an anti-oxidant.
(28). A composition comprising:
   a) an agent capable of increasing intracellular or intramitochondrial concentration of NAD in a subject;
   b) an agent capable of increasing glutathione concentration in the subject; and
   c) alpha-lipoic acid or a precursor to alpha-lipoic acid.
(29). A composition comprising:
   a) an agent capable of increasing intracellular or intramitochondrial concentration of NAD in a subject;
   b) an agent capable of increasing glutathione concentration in the subject; and
   c) an agent capable of increasing the level of Coenzyme A in the subject.
(30). A composition comprising:
   a) an agent capable of increasing intracellular or intramitochondrial concentration of NAD in a subject;
   b) an agent capable of increasing glutathione concentration in the subject;
   c) an anti-oxidant; and
   d) alpha-lipoic acid or a precursor to alpha-lipoic acid.
(31). A composition comprising:
   a) an agent capable of increasing intracellular or intramitochondrial concentration of NAD in a subject;
   b) an agent capable of increasing glutathione concentration in the subject;
   c) an anti-oxidant; and
   d) an agent capable of increasing the level of Coenzyme A in the subject.
(32). A composition comprising:
   a) an agent capable of increasing intracellular or intramitochondrial concentration of NAD in a subject;
   b) an agent capable of increasing glutathione concentration in the subject;
   c) alpha-lipoic acid or a precursor to alpha-lipoic acid; and
   d) an agent capable of increasing the level of Coenzyme A in the subject.
(33). The composition of any of (24)-(32), wherein said agents are present in the composition in an amount effective to prevent or reduce the alcohol flush reaction in the subject.
(34). A composition for use by a subject to prevent or reduce the alcohol flush reaction, said composition comprising:
   a) an agent capable of increasing intracellular or intramitochondrial concentration of NAD in the subject;
   b) an agent capable of increasing glutathione concentration in the subject;
   c) an agent which is an anti-oxidant; and
   d) an agent which is alpha-lipoic acid or a precursor to alpha-lipoic acid; and
   e) an agent capable of increasing the level of Coenzyme A in the subject.
(35). The composition of any of (24)-(33) or the composition for use of (34), wherein said agents are present in the composition in an amount effective to prevent or reduce the alcohol flush reaction in a subject.
(36). The composition for use of any of (1)-(11) or (34), further comprising an agent capable of raising the blood level of all-trans retinoic acid.
(37). The composition of any of (24)-(33), further comprising an agent capable of raising the blood level of all-trans retinoic acid.
(38). The method or use of any of (12)-(23), wherein the composition, supplement, or medicament further comprises an agent capable of raising the blood level of all-trans retinoic acid.
(39). A composition comprising: a) an agent selected from the group consisting of nicotinamide, nicotinamide riboside, niacin, and oxaloacetate; and b) an agent selected from the group consisting of N-acetyl-cysteine, Vitamin C, alpha lipoic acid, L-cysteine, glutathione, glycine, L-glutamic acid, adenosylmethionine, and conjugated linoleic acid; wherein said agents are present in the composition in an amount sufficient to prevent or reduce the alcohol flush reaction in the subject.
(40). The composition of (39), further comprising an agent selected from the group consisting of pantothenic acid, pantethine, pantetheine, 4'-phosphopantothenate, and 4'-phosphopantetheine.
(41). The composition of (39) or (40), further comprising an agent which is selected from the group consisting of Vitamin C, Vitamin E, and alpha-lipoic acid.
(42). The composition of (39) or (40), further comprising alpha-lipoic acid or a precursor to alpha-lipoic acid.
(43). The composition of any one of (39)-(42), further comprising an agent selected from the group consisting of beta-carotene and a retinol ester.
(44). The composition of (43), wherein the agent is a retinol ester selected from the group consisiting of retinyl arachidonate, retinyl stearate, retinyl oleate, retinyl linoleate, retinyl heptadecanoate, retinyl palmitate, retinyl myristate, retinyl laurate, retinyl caprylate, and retinyl acetate.
(45). A composition comprising an agent capable of increasing intracellular or intramitochondrial concentration of NAD in a subject; and *Hovenia dulcis* fruit extract.
(46). A composition comprising an agent capable of increasing intracellular or intramitochondrial concentration of NAD in a subject; and *Silybum marianum* extract.
(47). A composition comprising an agent capable of increasing intracellular or intramitochondrial concentration of NAD in a subject; *Hovenia dulcis* fruit extract; and *Silybum marianum* extract.
(48). A composition comprising: a) an agent selected from the group consisting of nicotinamide, nicotinamide riboside, niacin, and oxaloacetate; and b) one or more, and preferably, 3, 4, 5 or more, agents selected from the group consisting of *Hovenia dulcis* fruit extract, *Silybum marianum* extract, N-acetyl-cysteine, Vitamin C, alpha lipoic acid, L-cysteine, glutathione, glycine, L-glutamic acid, adenosylmethionine, and conjugated linoleic acid; wherein said agents are present in the composition in an amount sufficient to prevent or reduce the alcohol flush reaction in the subject.
(49). A composition comprising: a) an agent selected from the group consisting of nicotinamide, nicotinamide riboside, niacin, and oxaloacetate; b) *Hovenia dulcis* fruit extract; c) *Silybum marianum* extract; and d) α-lipoic acid; wherein said agents are present in the composition in an amount sufficient to prevent or reduce the alcohol flush reaction in the subject.
(50). A composition comprising: a) an agent selected from the group consisting of nicotinamide, nicotinamide riboside, niacin, and oxaloacetate; b) *Hovenia dulcis* fruit extract; c) *Silybum marianum* extract; d) vitamin A; e) vitamin B5; f) vitamin c; g) N-acetyl cysteine; h) thiamine; and i) α-lipoic acid; wherein said agents are present in the composition in an amount sufficient to prevent or reduce the alcohol flush reaction in the subject.
(51). A composition as described herein in (45)-(50), comprising at least 500 mg niacinamide.
(52). Any of the compositions, compositions for use, or methods of (1)-(50), wherein the composition, supplement, dosage, or medicament is in tablet or capsule form.
(53). Any of the compositions, compositions for use, or methods of (1)-(50), wherein the composition, supplement, dosage, or medicament is in liquid or beverage form.
(54). Any of the compositions, compositions for use, or methods of (1)-(50), wherein the composition, supplement, dosage, or medicament is provided in a food.

Although the foregoing invention has been described in some detail by way of illustration and example for purposes of clarity of understanding, it is apparent to those skilled in the art that certain changes and modifications will be practiced.

## Claims

1. A composition for use by a subject to prevent or reduce the alcohol flush reaction, said composition comprising:
(a) an agent selected from nicotinamide, nicotinamide riboside, niacin, and oxaloacetate; and
(b) an agent selected from N-acetyl-cysteine, Vitamin C, alpha lipoic acid, L-cysteine, glutathione, glycine, L-glutamic acid, adenosylmethionine, and conjugated linoleic acid; and
(c) an agent selected from pantothenic acid, pantethine, pantetheine, 4'-phosphopantothenate, and 4'-phosphopantetheine;
wherein the composition is administered daily for at least 30 days prior to consumption of alcohol by the subject;
wherein said agents are present in the composition in an amount sufficient to prevent or reduce the alcohol flush reaction in the subject.

2. A composition for use according to claim 1, further comprising an agent which is selected from Vitamin C, Vitamin E, and alpha-lipoic acid.

3. A composition for use according to claim 1 or 2, further comprising alpha-lipoic acid.

4. A composition for use according to any one of claims 1 to 3, further comprising an agent selected from beta-carotene and a retinol ester.

5. A composition for use according to claim 4, wherein the agent is a retinol ester selected from retinyl arachidonate, retinyl stearate, retinyl oleate, retinyl linoleate, retinyl heptadecanoate, retinyl palmitate, retinyl myristate, retinyl laurate, retinyl caprylate, and retinyl acetate.

6. A composition for use according to claim 4, wherein the agent is beta-carotene.

7. A composition for use according to any one of claims 1 to 6, further comprising thiamine.

8. A composition for use according to claim 1, comprising:
(i) between 300 mg and 1.5 g nicotinamide,
(ii) between 10 mg and 500 mg pantothenic acid or a salt thereof, and
(iii) between 100 mg and 600 mg N-acetylcysteine,
and an excipient acceptable for daily oral administration.

9. A composition for use according to claim 8, further comprising vitamin C in an amount between 100 mg and 500 mg.

10. A composition for use according to claim 8 or 9, further comprising alpha-lipoic acid in an amount between 50 mg and 250 mg.

11. A composition for use according to any one of claims 8 to 10, further comprising beta-carotene in an amount between 100 IU and 15000 IU.

12. A composition for use according to any one of claims 8 to 11, further comprising thiamine.

13. A composition for use according to any one of claims 8 to 11, further comprising thiamine in an amount between 2.5 mg and 10 mg.

14. A composition for use according to claim 8, further comprising one or more of vitamin A, vitamin C, thiamine, and α-lipoic acid.

15. A composition for use according to claim 8, further comprising one or more of:
1250 IU to 2500 IU vitamin A,
75 mg to 150 mg vitamin C,
2.5 to 10 mg thiamine, and
62 to 125 mg α-lipoic acid.

16. A composition for use according to claim 14 or 15, comprising each of vitamin A, vitamin C, thiamine, and α-lipoic acid.

17. A composition for use of any one of claims 1 to 16, wherein the composition is provided as a tablet or a capsule.

18. A composition for use of any one of claims 1 to 16, wherein the composition is provided in a food or in a beverage.

## Patentansprüche

1. Zusammensetzung zur Verwendung von einem Individuum zur Vorbeugung oder Verringerung von Alkoholintoleranz, wobei die Zusammensetzung Folgendes umfasst:
(a) ein Mittel, das aus Nicotinamid, Nicotinamidribosid, Niacin und Oxaloacetat ausgewählt ist; und
(b) ein Mittel, das aus N-Acetylcystein, Vitamin C, α-Liponsäure, L-Cystein, Glutathion, Glycin, L-Glutaminsäure, Adenosylmethionin und konjugierter Linolsäure ausgewählt ist; und
(c) ein Mittel, das aus Pantothensäure, Pantethin, Pantethein, 4'-Phosphopantothenat und 4'-Phosphopantethein ausgewählt ist;
wobei die Zusammensetzung täglich für zumindest 30 Tage vor der Konsumation von Alkohol durch das Individuum verabreicht wird;
wobei die Mittel in der Zusammensetzung in einer Menge vorhanden sind, die ausreicht, um die Alkoholintoleranzreaktion beim Individuum zu verhindern oder zu verringern.

2. Zusammensetzung zur Verwendung nach Anspruch 1, die weiters ein Mittel umfasst, das aus Vitamin C, Vitamin E und α-Liponsäure ausgewählt ist.

3. Zusammensetzung zur Verwendung nach Anspruch 1 oder 2, das weiters α-Liponsäure umfasst.

4. Zusammensetzung zur Verwendung nach einem der Ansprüche 1 bis 3, das weiters ein Mittel umfasst, das aus β-Carotin und einem Retinolester ausgewählt ist.

5. Zusammensetzung zur Verwendung nach Anspruch 4, wobei das Mittel ein Retinolester ist, der aus Retinylarachidonat, Retinylstearat, Retinyloleat, Retinyllinoleat, Retinylheptadecanoat, Retinylpalmitat, Retinylmyristat, Retinyllaurat, Retinylcaprylat und Retinylacetat ausgewählt ist.

6. Zusammensetzung zur Verwendung nach Anspruch 4, wobei das Mittel β-Carotin ist.

7. Zusammensetzung zur Verwendung nach einem der Ansprüche 1 bis 6, das weiters Thiamin umfasst.

8. Zusammensetzung zur Verwendung nach Anspruch 1, die Folgendes umfasst:
(i) zwischen 300 mg und 1,5 g Nicotinamid,
(ii) zwischen 10 mg und 500 mg Pantothensäure oder ein Salz davon und
(iii) zwischen 100 mg und 600 mg N-Acetylcystein
und einen zur täglichen oralen Verabreichung geeigneten Exzipienten.

9. Zusammensetzung zur Verwendung nach Anspruch 8, die weiters Vitamin C in einer Menge zwischen 100 mg und 500 mg umfasst.

10. Zusammensetzung zur Verwendung nach Anspruch 8 oder 9, die weiters α-Liponsäure in einer Menge zwischen 50 mg und 250 mg umfasst

11. Zusammensetzung zur Verwendung nach einem der Ansprüche 8 bis 10, die weiters β-Carotin in einer Menge zwischen 100 IU und 15000 IU umfasst.

12. Zusammensetzung zur Verwendung nach einem der Ansprüche 8 bis 11, die weiters Thiamin umfasst.

13. Zusammensetzung zur Verwendung nach einem der Ansprüche 8 bis 11, die weiters Thiamin in einer Menge zwischen 2,5 mg und 10 mg umfasst.

14. Zusammensetzung zur Verwendung nach Anspruch 8, die weiters eines oder mehrere aus Vitamin A, Vitamin C, Thiamin und a-Liponsäure umfasst.

15. Zusammensetzung zur Verwendung nach Anspruch 8, die weiters eines oder mehrere aus Folgenden umfasst:
1250 IU bis 2500 IU Vitamin A,
75 mg bis 150 mg Vitamin C,
2,5 bis 10 mg Thiamin und
62 bis 125 mg α-Liponsäure.

16. Zusammensetzung zur Verwendung nach Anspruch 14 oder 15, die jedes aus Vitamin A, Vitamin C, Thiamin und a-Liponsäure umfasst.

17. Zusammensetzung zur Verwendung nach einem der Ansprüche 1 bis 16, wobei die Zusammensetzung als Tablette oder Kapsel bereitgestellt ist.

18. Zusammensetzung zur Verwendung nach einem der Ansprüche 1 bis 16, wobei die Zusammensetzung in einem Nahrungsmittel oder Getränk bereitgestellt ist.

## Revendications

1. Composition pour une utilisation par un sujet pour prévenir ou réduire les bouffées associées à la consommation d'alcool, ladite composition comprenant :
(a) un agent choisi parmi le nicotinamide, le nicotinamide riboside, la niacine et l'oxaloacétate ; et
(b) un agent choisi parmi la N-acétyl-cystéine, la vitamine C, l'acide alpha lipoïque, la L-cystéine, la glutathione, la glycine, l'acide L-glutamique, l'adénosylméthionine et l'acide linoléique conjugué ; et
(c) un agent choisi parmi l'acide pantothénique, la pantéthine, la pantéthéine, le 4'-phosphopantothénate et la 4'-phosphopantéthéine ;
dans laquelle la composition est administrée quotidiennement pendant au moins 30 jours avant la consommation d'alcool par le sujet ;
dans laquelle lesdits agents sont présents dans la composition en une quantité suffisante pour prévenir ou réduire les bouffées associées à la consommation d'alcool chez le sujet.

2. Composition pour une utilisation selon la revendication 1, comprenant en outre un agent qui est choisi parmi la vitamine C, la vitamine E et l'acide alpha-lipoïque.

3. Composition pour une utilisation selon la revendication 1 ou 2, comprenant en outre de l'acide alpha-lipoïque.

4. Composition pour une utilisation selon l'une quelconque des revendications 1 à 3, comprenant en outre un agent choisi parmi le bêta-carotène et un ester de rétinol.

5. Composition pour une utilisation selon la revendication 4, dans laquelle l'agent est un ester de rétinol choisi parmi l'arachidonate de rétinyle, le stéarate de rétinyle, l'oléate de rétinyle, le linoléate de rétinyle, l'heptadécanoate de rétinyle, le palmitate de rétinyle, le myristate de rétinyle, le laurate de rétinyle, le caprylate de rétinyle et l'acétate de rétinyle.

6. Composition pour une utilisation selon la revendication 4, dans laquelle l'agent est le bêta-carotène.

7. Composition pour une utilisation selon l'une quelconque des revendications 1 à 6, comprenant en outre de la thiamine.

8. Composition pour une utilisation selon la revendication 1, comprenant :
(i) entre 300 mg et 1,5 g de nicotinamide,
(ii) entre 10 mg et 500 mg d'acide pantothénique ou d'un sel de celui-ci, et
(iii) entre 100 mg et 600 mg de N-acétylcystéine,
et un excipient acceptable pour une administration orale quotidienne.

9. Composition pour une utilisation selon la revendication 8, comprenant en outre de la vitamine C en une quantité entre 100 mg et 500 mg.

10. Composition pour une utilisation selon la revendication 8 ou 9, comprenant en outre de l'acide alpha-lipoïque en une quantité entre 50 mg et 250 mg.

11. Composition pour une utilisation selon l'une quelconque des revendications 8 à 10, comprenant en outre du bêta-carotène en une quantité entre 100 UI et 15 000 UI.

12. Composition pour une utilisation selon l'une quelconque des revendications 8 à 11, comprenant en outre de la thiamine.

13. Composition pour une utilisation selon l'une quelconque des revendications 8 à 11, comprenant en outre de la thiamine en une quantité entre 2,5 mg et 10 mg.

14. Composition pour une utilisation selon la revendication 8, comprenant en outre un ou plusieurs de la vitamine A, de la vitamine C, de la thiamine et d'un acide α-lipoïque.

15. Composition pour une utilisation selon la revendication 8, comprenant en outre un ou plusieurs de :
1 250 UI à 2 500 UI de vitamine A,
75 mg à 150 mg de vitamine C,
2,5 à 10 mg de thiamine, et
62 à 125 mg d'acide α-lipoïque.

16. Composition pour une utilisation selon la revendication 14 ou 15, comprenant chacune de la vitamine A, de la vitamine C, de la thiamine et un acide α-lipoïque.

17. Composition pour une utilisation selon l'une quelconque des revendications 1 à 16, dans laquelle la composition se présente sous la forme d'un comprimé ou d'une capsule.

18. Composition pour une utilisation selon l'une quelconque des revendications 1 à 16, dans laquelle la composition se présente dans un aliment ou dans une boisson.
